# EUROPEAN PATENT SPECIFICATION

(11) **EP 3 089 990 B1**
(45) Date of publication and mention of the grant of the patent: **20.05.2020**
(21) Application number: 14821373.9
(22) Date of filing: 05.12.2014
(51) Int. Cl.: C07K 14/32, C12N 15/75

(54) **ENHANCED PROTEIN EXPRESSION**
ERHÖHTE PROTEINEXPRESSION
EXPRESSION AMÉLIORÉE DE PROTÉINE

(30) Priority: 30.12.2013 US 201361922038 P
(43) Date of publication of application: 09.11.2016
(73) Proprietor: Danisco US Inc., Palo Alto, CA 94304 (US)
(72) Inventor: BONGIORNI, Cristina, Palo Alto, California 94304 (US); SCHMIDT, Brian F., Palo Alto, California 94304 (US); VAN KIMMENADE, Anita, Palo Alto, California 94304 (US)
(74) Representative: Mewburn Ellis LLP
(86) International application number: PCT/US2014/068745
(87) International publication number: WO 2015/102809

(56) References cited:
- WO-A2-2005/069762
- WO-A2-2008/066931
- JP-A- 2009 538 603
- H. GAO ET AL: "The E1 and E2 Subunits of the Bacillus subtilis Pyruvate Dehydrogenase Complex Are Involved in Regulation of Sporulation", JOURNAL OF BACTERIOLOGY, vol. 184, no. 10, 15 May 2002 (2002-05-15) , pages 2780-2788, XP055172369, ISSN: 0021-9193, DOI: 10.1128/JB.184.10.2780-2788.2002
- Hui Xu ET AL: "Development of a mutant strain of Bacillus subtilis showing enhanced production of acetoin", African Journal of Biotechnology, 31 January 2011 (2011-01-31), pages 779-788, XP055175180, Victoria Island DOI: 10.5897/AJB10.1455 Retrieved from the Internet: URL:http://search.proquest.com/docview/166 0383940
- Anonymous: "Hit details for EM_PAT:FV536035", , 12 November 2009 (2009-11-12), XP055394250, Retrieved from the Internet: URL:http://ibis/exam/hitDetails.jsp?id=967 96547 [retrieved on 2017-07-27]

## Description

### FIELD OF THE INVENTION

The present invention relates in general to bacterial cells having a genetic alteration that results in increased expression of a protein of interest and methods of making and using such cells. Aspects of the present invention include Gram-positive microorganisms, such as *Bacillus* species, having a genetic alteration that reduces the expression of a gene in the *pdh* operon and results in enhanced expression of a protein of interest.

### BACKGROUND OF THE INVENTION

Genetic engineering has allowed the improvement of microorganisms used as industrial bioreactors, cell factories and in food fermentations. Gram-positive organisms, including a number of *Bacillus* species, are used to produce a large number of useful proteins and metabolites (see, e.g., Zukowski, "Production of commercially valuable products," In: Doi and McGlouglin (eds.) Biology of Bacilli: Applications to Industry, Butterworth-Heinemann, Stoneham. Mass pp 311-337 [1992]). Common *Bacillus* species used in industry include *B*. *licheniformis, B. amyloliquefaciens* and *B. subtilis.* Because of their GRAS (generally recognized as safe) status, strains of these *Bacillus* species are natural candidates for the production of proteins utilized in the food and pharmaceutical industries. Examples of proteins produced in Gram-positive organisms include enzymes, e.g., α-amylases, neutral proteases, and alkaline (or serine) proteases.

In spite of advances in the understanding of production of proteins in bacterial host cells, there remains a need for to develop new recombinant strains that express increased levels of a protein of interest.

### SUMMARY OF THE INVENTION

The present invention provides recombinant Gram positive cells that express increased levels of a protein of interest and methods of making and using the same. In particular, the present invention relates to bacterial cells having a genetic alteration that results in increased expression of a protein of interest as compared to bacterial cells that do not have the genetic alteration. Aspects of the present invention include Gram-positive microorganisms, such as *Bacillus* species, having a genetic alteration that reduces the expression of a gene in the *pdh* operon and results in enhanced expression of a protein of interest. Methods of making and using such recombinant bacterial cells are also provided.
Aspects of the invention include a method for increasing expression of a protein of interest from a Gram positive bacterial cell comprising a) obtaining an altered Gram positive bacterial cell capable of producing a protein of interest, wherein said altered Gram positive bacterial cell comprises at least one genetic alteration that reduces expression of the *pdhA* gene as compared to the expression of the *pdhA* gene in a corresponding unaltered Gram positive bacterial cell grown under essentially the same culture conditions and/or reduces expression of the *pdhB* gene as compared to the expression of the *pdhB* gene in a corresponding unaltered Gram positive bacterial cell grown under essentially the same culture conditions wherein said genetic alteration is in the *pdhD* gene of the *pdh* operon; and b) culturing said altered Gram positive bacterial cell under conditions such that said protein of interest is expressed by said altered Gram positive bacterial cell, wherein expression of said protein of interest is increased in said altered Gram positive bacterial cell compared to the expression of said protein of interest in a corresponding unaltered Gram positive bacterial cell grown under essentially the same culture conditions.

In certain embodiments, the altered Gram positive bacterial cell is a *Bacillus sp.* strain (e.g., *Bacillus sp.* strain is selected from the group consisting of: *B. licheniformis, B. lentus, B. subtilis, B*. amyloliquefaciens, *B. brevis, B. stearothermophilus, B. alkalophilus, B. coagulans, B. circulans, B. pumilus, B. lautus, B. clausii, B. megaterium,* and *B*. *thuringiensis*). In certain embodiments, the *Bacillus sp.* strain is a *B. subtilis* strain. In certain embodiments, the altered Gram positive bacterial cell further comprises a mutation in a gene selected from the group consisting of *degU, degQ, degS, scoC4, spollE,* and *oppA.* In certain embodiments, the mutation is *degU(Hy)32.*

In certain embodiments, the altered Gram positive bacterial cell has reduced expression of the *pdhA* gene as compared to the expression of the *phdA* gene in a corresponding unaltered Gram positive bacterial cell grown under essentially the same culture conditions. In certain embodiments, the altered Gram positive bacterial cell has reduced expression of the *pdhB* gene as compared to the expression of the *phdB* gene in a corresponding unaltered Gram positive bacterial cell grown under essentially the same culture conditions. In certain embodiments, the altered Gram positive bacterial cell has reduced expression of the *pdhA* gene and the *pdhB* gene as compared to the expression of the *phdA* gene and the *pdhB* gene in a corresponding unaltered Gram positive bacterial cell grown under essentially the same culture conditions.

In certain embodiments, the genetic alteration results in a decrease in the level of an mRNA transcript derived from the *pdh* operon in the altered Gram positive bacterial cell as compared to a corresponding unaltered Gram positive bacterial cell grown under essentially the same culture conditions.

In certain embodiments, the mutation is in the *pdhD* gene of said *pdh* operon. In certain embodiments, the *pdhD* gene is at least 60% identical to SEQ ID NO:1. In certain embodiments, the genetic alteration is a silent mulation. In certain embodiments, the mutation is at a nucleotide position corresponding to nucleotide 729 of SEQ ID NO: 1. In certain embodiments, the mutation is a C to T mutation at a nucleotide position corresponding to nucleotide 729 of SEQ ID NO:1.

In certain embodiments, the protein of interest is a homologous protein. In certain embodiments, the protein of interest is a heterologous protein. In certain embodiments, the protein of interest is an enzyme. In certain embodiments, the enzyme is selected from the group consisting of: protease, cellulase, pullulanase, amylase, carbohydrase, lipase, isomerase, transferase, kinase, and phosphatase. In certain embodiments, the protein of interest is a protease. In certain embodiments, the protease is a subtilisin. In certain embodiments, the subtilisin is selected from the group consisting of: subtilisin 168, subtilisin BPN', subtilisin Carlsberg, subtilisin DY, subtilisin 147, subtilisin 309, and variants thereof.

In certain embodiments, the method further comprisies recovering said protein of interest.

Aspects of the present invention include an altered Gram positive bacterial cell, wherein said altered Gram positive bacterial cell comprises at least one genetic alteration that reduces expression of one or more genes in the *pdh* operon as compared to a corresponding unaltered Gram positive bacterial cell grown under essentially the same culture conditions. In certain embodiments, the altered Gram positive bacterial cell is a *Bacillus sp.* strain. In certain embodiments, the *Bacillus sp.* strain is selected from the group consisting of: *B. licheniformis, B. lentus, B. subtilis, B*. amyloliquefaciens, *B. brevis, B. stearothermophilus, B. alkalophilus, B. coagulans, B. circulans, B. pumilus, B. lautus, B. clausii, B. megaterium,* and *B*. *thuringiensis.* In certain embodiments, the *Bacillus sp.* strain is a *B*. *subtilis* strain. In certain embodiments, the altered Gram positive bacterial cell further comprises a mutation in a gene selected from the group consisting of *degU, degQ, degS, scoC4, spollE,* and *oppA.* In certain embodiments, the mutation is *degU(Hy)32.*

In certain embodiments, the altered Gram positive bacterial cell has reduced expression of the *pdhA* gene as compared to the expression of the *phdA* gene in a corresponding unaltered Gram positive bacterial cell grown under essentially the same culture conditions. In certain embodiments, the altered Gram positive bacterial cell has reduced expression of the *pdhB* gene as compared to the expression of the *phdB* gene in a corresponding unaltered Gram positive bacterial cell grown under essentially the same culture conditions. In certain embodiments, the altered Gram positive bacterial cell has reduced expression of the *pdhA* gene and the *pdhB* gene as compared to the expression of the *phdA* gene and the *pdhB* gene in a corresponding unaltered Gram positive bacterial cell grown under essentially the same culture conditions. In certain embodiments, the genetic alteration results in a decrease in the level of an mRNA transcript derived from the *pdh* operon in the altered Gram positive bacterial cell as compared to a corresponding unaltered Gram positive bacterial cell grown under essentially the same culture conditions.

In certain embodiments, the mutation is in the *pdhD* gene of said *pdh* operon. In certain embodiments, the *pdhD* gene is at least 60% identical to SEQ ID NO:1. In certain embodiments, the genetic alteration is a silent mulation. In certain embodiments, the mutation is at a nucleotide position corresponding to nucleotide 729 of SEQ ID NO: 1. In certain embodiments, the mutation is a C to T mutation at a nucleotide position corresponding to nucleotide 729 of SEQ ID NO:1 (shown in SEQ ID NO:3). In certain embodiments, the altered cell expresses a protein of interest. In certain embodiments, the protein of interest is a homologous protein. In certain embodiments, the protein of interest is a heterologous protein. In certain embodiments, the protein of interest is an enzyme.

In certain embodiments, the enzyme is selected from the group consisting of: protease, cellulase, pullulanase, amylase, carbohydrase, lipase, isomerase, transferase, kinase, and phosphatase. In certain embodiments, the protein of interest is a protease. In certain embodiments, the protease is a subtilisin In certain embodiments, the subtilisin is selected from the group consisting of: subtilisin 168, subtilisin BPN', subtilisin Carlsberg, subtilisin DY, subtilisin 147, subtilisin 309, and variants thereof.

Also described is a method for obtaining an altered Gram positive bacterial cell with improved protein production capability comprising introducing at least one genetic alteration into a parental Gram positive bacterial cell that reduces the expression level of one or more genes in the *pdh* operon. In certain embodiments, the altered Gram positive bacterial cell is a *Bacillus sp.* strain. In certain embodiments, the *Bacillus sp.* strain is selected from the group consisting of: *B. licheniformis, B. lentus, B. subtilis, B*. amyloliquefaciens, *B. brevis, B. stearothermophilus, B. alkalophilus, B. coagulans, B. circulans, B. pumilus, B. lautus, B. clausii, B. megaterium,* and *B. thuringiensis.* In certain embodiments, the *Bacillus sp.* strain is a *B. subtilis* strain. In certain embodiments, the altered Gram positive bacterial cell further comprises a mutation in a gene selected from the group consisting of *degU, degQ, degS, scoC4, spollE,* and *oppA.* In certain embodiments, the mutation is *degU(Hy)32.*

The altered Gram positive bacterial cell may have reduced expression of the *pdhA* gene as compared to the expression of the *phdA* gene in said parental Gram positive bacterial cell grown under essentially the same culture conditions. The altered Gram positive bacterial cell may have reduced expression of the *pdhB* gene as compared to the expression of the *phdB* gene in said parental Gram positive bacterial cell grown under essentially the same culture conditions. The altered Gram positive bacterial cell may have reduced expression of the *pdhA* gene and the *pdhB* gene as compared to the expression of the *phdA* gene and the *pdhB* gene in said parental Gram positive bacterial cell grown under essentially the same culture conditions.

The genetic alteration may result in a decrease in the level of an mRNA transcript derived from the *pdh* operon in the altered Gram positive bacterial cell as compared to said parental Gram positive bacterial cell grown under essentially the same culture conditions. The mutation may be in the *pdhD* gene of said *pdh* operon. The *pdhD* gene is at least 60% identical to SEQ ID NO:1. The genetic alteration may be a silent mulation. The mutation may be at a nucleotide position corresponding to nucleotide 729 of SEQ ID NO: 1. The mutation may be a C to T mutation at a nucleotide position corresponding to nucleotide 729 of SEQ ID NO:1 (shown in SEQ ID NO:3).

The said altered Gram positive bacterial cell may express a protein of interest. The method may further comprise introducing an expression cassette encoding said protein of interest into said parental Gram positive bacterial cell. The method may further comprise introducing an expression cassette encoding said protein of interest into said altered Gram positive bacterial cell. The protein of interest may be a homologous protein. The protein of interest may be a heterologous protein. The protein of interest may be enzyme. The enzyme may be selected from the group consisting of: protease, cellulase, pullulanase, amylase, carbohydrase, lipase, isomerase, transferase, kinase, and phosphatase. In certain embodiments, the protein of interest is a protease. The protease may be a subtilisin. The subtilisin may be selected be from the group consisting of: subtilisin 168, subtilisin BPN', subtilisin Carlsberg, subtilisin DY, subtilisin 147, subtilisin 309, and variants thereof.

The method may further comprise culturing said altered Gram positive bacterial cell under conditions such that said protein of interest is expressed by said altered Gram positive bacterial cell. The method may further comprise recovering said protein of interest.

Also contemplated is an altered Gram positive bacterial cell produced by the methods described above.

Aspects of the present invention include a polynucleotide comprising a variant sequence derived from the *pdhD* gene, wherein said variant sequence:
derived from the *pdhD* gene, wherein said variant sequence is:
(a) at least 30 nucleotides in length and at least 95% identical to all or a part of SEQ ID NO: 1, or
(a) at least 100 nucleotides in length and at least 90% identical to all or a part of SEQ ID NO: 1; or
(b) at least 500 nucleotides in length and at least 85% identical to all or a part of SEQ ID NO: 1;
and comprises at least one mutation at a nucleotide position in the *pdhD* gene that leads to reduced expression of the *pdhA* gene and/or the *pdhB* gene when said at least one mutation is present in the endogenous *pdhD* gene of a Gram positive bacterial cell, wherein said at least one mutation is a silent mutation at a nucleotide position corresponding to nucleotide 729 of SEQ ID NO: 1, wherein said mutation is a C to T mutation at a nucleotide position corresponding to nucleotide 729 of SEQ ID NO: 1. In some embodiments the variant sequence is at least 90% identical to all or a part of SEQ ID NO:3. In certain embodiments, the variant sequence is identical to all or a part of SEQ ID NO:3. In certain embodiments, the variant sequence is at least 20 nucleotides in length. In certain embodiments, the variant sequence is at least 50 nucleotides in length. In certain embodiments, the variant sequence is at least 200 nucleotides in length.

Aspects of the present invention include a vector comprising the polynucleotide sequence as described above. In certain embodiments, the vector is a targeting vector designed to introduce the at least one mutation in said polynucleotide sequence into the corresponding location in the *pdh* operon of a Gram positive bacterial cell by homologous recombination when transformed into said Gram positive bacterial cell.

Aspects of the present invention include a method for enhancing expression of a protein of interest in a Gram positive bacterial cell comprising:
a) transforming a parental Gram positive bacterial cell with the vector above;
b) allowing homologous recombination of said vector and the corresponding region in the *pdh* operon of said parental Gram positive bacterial cell to produce an altered Gram positive bacterial cell; and
c) growing said altered Gram positive bacterial cell under conditions suitable for the expression of said protein of interest, wherein the production of said protein of interest is increased in the altered Gram positive bacterial cell as compared to said Gram positive bacterial cell prior to said transformation in step.

In certain embodiments, the parental Gram positive bacterial cell is a *Bacillus sp.* strain. In certain embodiments, the *Bacillus sp.* strain is selected from the group consisting of: *B*. *licheniformis, B. lentus, B. subtilis, B*. amyloliquefaciens, *B. brevis, B. stearothermophilus, B. alkalophilus, B. coagulans, B. circulans, B. pumilus, B. lautus, B. clausii, B. megaterium,* and *B*. *thuringiensis.* In certain embodiments, the *Bacillus sp.* strain is a *B*. *subtilis* strain. In certain embodiments, the altered Gram positive bacterial cell further comprises a mutation in a gene selected from the group consisting of *degU, degQ, degS, scoC4, spollE,* and *oppA.* In certain embodiments, the mutation is *degU(Hy)32.*

In certain embodiments, the altered Gram positive bacterial cell has reduced expression of the *pdhA* gene as compared to the expression of the *phdA* gene in said parental Gram positive bacterial cell grown under essentially the same culture conditions. In certain embodiments, the altered Gram positive bacterial cell has reduced expression of the *pdhB* gene as compared to the expression of the *phdB* gene in said parental Gram positive bacterial cell grown under essentially the same culture conditions. In certain embodiments, the altered Gram positive bacterial cell has reduced expression of the *pdhA* gene and the *pdhB* gene as compared to the expression of the *phdA* gene and the *pdhB* gene in said parental Gram positive bacterial cell grown under essentially the same culture conditions. In certain embodiments, the genetic alteration results in a decrease in the level of an mRNA transcript derived from the *pdh* operon in the altered Gram positive bacterial cell as compared to said parental Gram positive bacterial cell grown under essentially the same culture conditions.

In certain embodiments, the mutation is in the *pdhD* gene of said *pdh* operon. In certain embodiments, the *pdhD* gene is at least 60% identical to SEQ ID NO:1. In certain embodiments, the genetic alteration is a silent mulation. In certain embodiments, the mutation is at a nucleotide position corresponding to nucleotide 729 of SEQ ID NO: 1. In certain embodiments, the mutation is a C to T mutation at a nucleotide position corresponding to nucleotide 729 of SEQ ID NO:1 (shown in SEQ ID NO:3). In certain embodiments, the protein of interest is a homologous protein. In certain embodiments, the protein of interest is a heterologous protein. In certain embodiments, the protein of interest is an enzyme. In certain embodiments, the enzyme is selected from the group consisting of: protease, cellulase, pullulanase, amylase, carbohydrase, lipase, isomerase, transferase, kinase, and phosphatase. In certain embodiments, the protein of interest is a protease. In certain embodiments, the protease is a subtilisin. In certain embodiments, the subtilisin is selected from the group consisting of: subtilisin 168, subtilisin BPN', subtilisin Carlsberg, subtilisin DY, subtilisin 147, subtilisin 309, and variants thereof.

In certain embodiments, the method further comprises recovering said protein of interest.

### BRIEF DESCRIPTION OF THE DRAWINGS

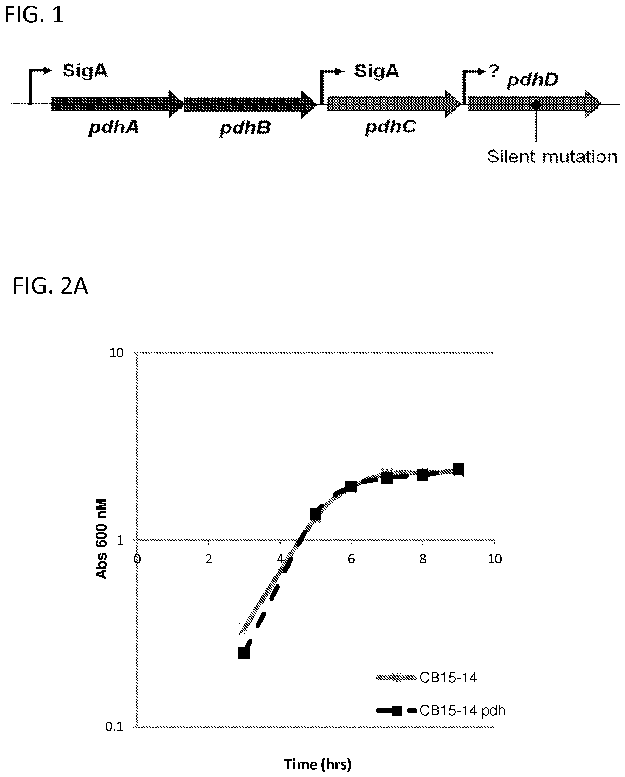

Figure 1 shows a schematic of the *pdh* operon from *Bacillus subtilis.* The location of the silent mutation described in the Examples is indicated. The *pdhA, pdhB, pdhC* and *pdhD* genes are indicated by arrows. Transcription initiation sites are shown as bent arrows. Transcription can continue from each transcription initiation site to the end of the operon, and thus transcripts that include more than one gene are possible (e.g., transcription from the first transcription initiation site can produce an mRNA encoding all 4 genes: *pdhABCD*)*.*

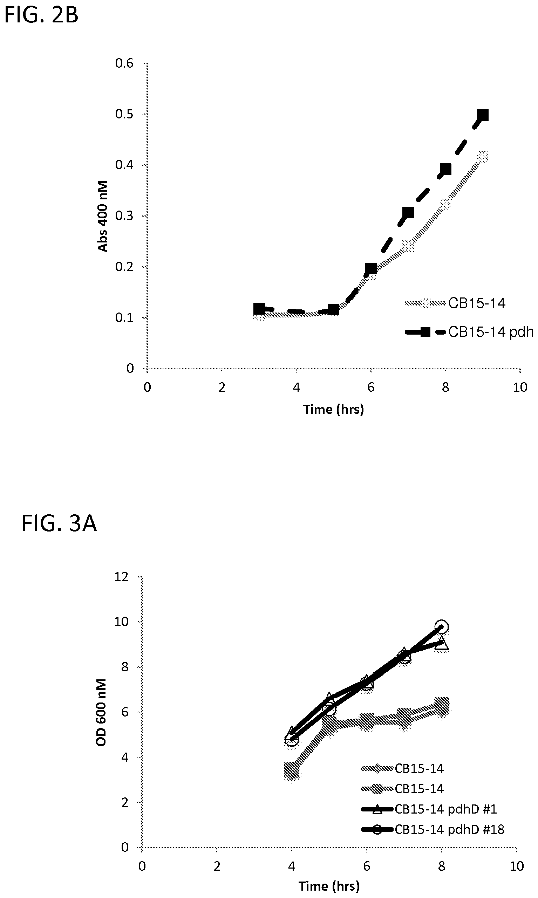

Figure 2A shows a graph of cell densities of AmyE expressing CB15-14 derivatives: CB15-14 (control strain) and CB15-14 pdh (strain containing the pdhD mutation). Figure 2B shows a graph of AmyE expression in CB15-14 derivatives: CB15-14 (control strain) and CB15-14 pdh (strain containing the pdhD mutation).

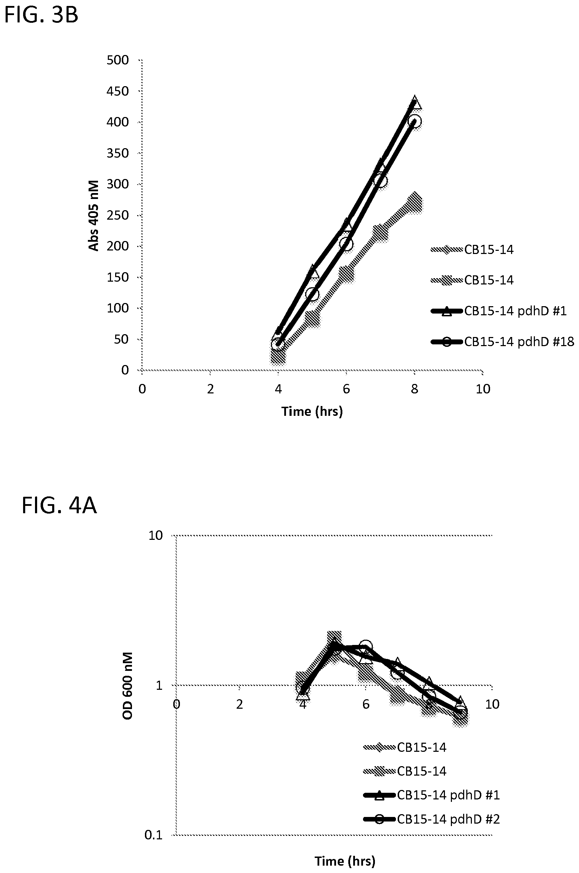

Figure 3A shows a graph of cell densities of FNA expressing CB15-14 derivatives: CB15-14 (control strains) and CB15-14 pdh #1 and #18 (strains containing the pdhD mutation). Figure 3B shows a graph of FNA expression in CB15-14 derivatives: CB15-14 (control strains) and CB15-14 pdh #1 and #18 (strains containing the pdhD mutation).

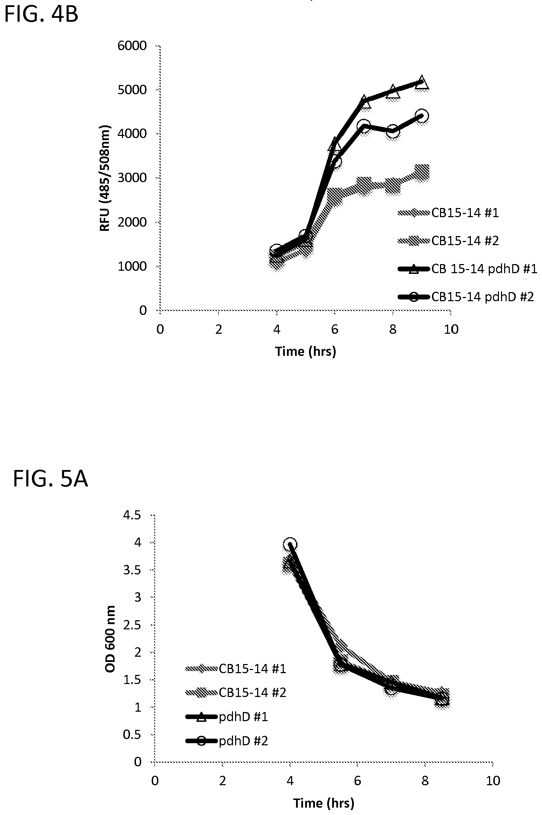

Figure 4A shows a graph of cell densities of GFP expressing CB15-14 derivatives: CB15-14 (control strains) and CB15-14 pdh #1 and #2 (strains containing pdhD mutation). Figure 4B shows a graph of GFP expression in CB15-14 derivatives: CB15-14 (control strains) and CB15-14 pdh #1 and #2 (strains containing pdhD mutation).

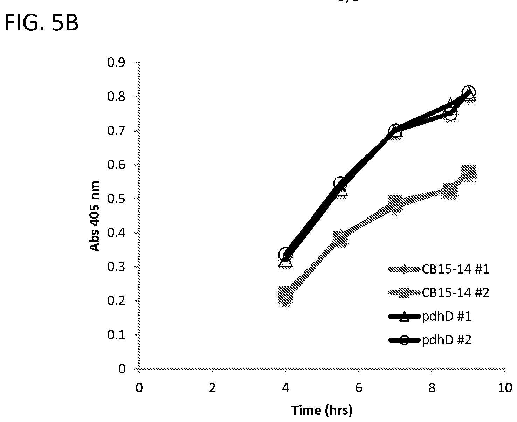

Figure 5A shows a graph of cell densities of BgIC expressing CB15-14 derivatives: CB15-14 #1 and #2 (control strains) and pdhD #1 and pdhD#2 (strains containing pdhD mutation). Figure 5B shows a graph of BgIC expression in CB15-14 derivatives: CB15-14 #1 and #2 (control strains) and pdhD #1 and pdhD#2 (strains containing pdhD mutation).

### DETAILED DESCRIPTION OF THE INVENTION

The present invention relates in general to bacterial cells having a genetic alteration that results in increased expression of a protein of interest and methods of making and using such cells. Aspects of the present invention include Gram-positive microorganisms, such as *Bacillus* species cells, having a genetic alteration that reduces the expression of a gene in the *pdh* operon which results in enhanced expression of a protein of interest.

Before the present compositions and methods are described in greater detail, it is to be understood that the present compositions and methods are not limited to particular embodiments described, as such may, of course, vary. It is also to be understood that the terminology used herein is for the purpose of describing particular embodiments only, and is not intended to be limiting, since the scope of the present compositions and methods will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the present compositions and methods. The upper and lower limits of these smaller ranges may independently be included in the smaller ranges and are also encompassed within the present compositions and methods, subject to any specifically excluded limit in the stated range. Where the stated range includes one or both of the limits, ranges excluding either or both of those included limits are also included in the present compositions and methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. For example, in connection with a numerical value, the term "about" refers to a range of -10% to +10% of the numerical value, unless the term is otherwise specifically defined in context. In another example, the phrase a "pH value of about 6" refers to pH values of from 5.4 to 6.6, unless the pH value is specifically defined otherwise.

The headings provided herein are not limitations of the various aspects or embodiments of the present compositions and methods which can be had by reference to the specification as a whole. Accordingly, the terms defined immediately below are more fully defined by reference to the specification as a whole.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art to which the present compositions and methods belongs. Although any methods and materials similar or equivalent to those described herein can also be used in the practice or testing of the present compositions and methods, representative illustrative methods and materials are now described.

The citation of any publication is for its disclosure prior to the filing date and should not be construed as an admission that the present compositions and methods are not entitled to antedate such publication by virtue of prior invention. Further, the dates of publication provided may be different from the actual publication dates which may need to be independently confirmed.

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is further noted that the term "consisting essentially of," as used herein refers to a composition wherein the component(s) after the term is in the presence of other known component(s) in a total amount that is less than 30% by weight of the total composition and do not contribute to or interferes with the actions or activities of the component(s).

It is further noted that the term "comprising," as used herein, means including, but not limited to, the component(s) after the term "comprising." The component(s) after the term "comprising" are required or mandatory, but the composition comprising the component(s) may further include other non-mandatory or optional component(s).

It is also noted that the term "consisting of," as used herein, means including, and limited to, the component(s) after the term "consisting of." The component(s) after the term "consisting of" are therefore required or mandatory, and no other component(s) are present in the composition.

Any recited method can be carried out in the order of events recited or in any other order which is logically possible.

### Definitions

As used herein, "host cell" refers to a cell that has the capacity to act as a host or expression vehicle for a newly introduced DNA sequence.

In certiain embodiments of the present invention, the host cells are bacterial cells, e.g., Gram-positive host cells *Bacillus sp.*

As used herein, "the genus *Bacillus"* or *"Bacillus sp."* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B*. *thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

As used herein, "nucleic acid" refers to a nucleotide or polynucleotide sequence, and fragments or portions thereof, as well as to DNA, cDNA, and RNA of genomic or synthetic origin which may be double-stranded or single-stranded, whether representing the sense or antisense strand. It will be understood that as a result of the degeneracy of the genetic code, a multitude of nucleotide sequences may encode a given protein.

As used herein, the term "vector" refers to any nucleic acid that can be replicated in cells and can carry new genes or DNA segments into cells. Thus, the term refers to a nucleic acid construct designed for transfer between different host cells. An "expression vector" refers to a vector that has the ability to incorporate and express heterologous DNA fragments in a foreign cell. Many prokaryotic and eukaryotic expression vectors are commercially available. A "targeting vector" is a vector that includes polynucleotide sequences that are homologus to a regin in the choromosome of a host cell into which it is transformed and that can drive homologous recombination at that region. Targetting vectors find use in introducing mutations into the chromosome of a cell through homologous recombination. In some embodiments, the targeting vector comprises comprises other non-homologous sequences, e.g., added to the ends *(i.e.,* stuffer sequences or flanking sequences). The ends can be closed such that the targeting vector forms a closed circle, such as, for example, insertion into a vector. Selection and/or construction of appropriate vectors is within the knowledge of those having skill in the art.

As used herein, the term "plasmid" refers to a circular double-stranded (ds) DNA construct used as a cloning vector, and which forms an extrachromosomal self-replicating genetic element in many bacteria and some eukaryotes. In some embodiments, plasmids become incorporated into the genome of the host cell.

By "purified" or "isolated" or "enriched" is meant that a biomolecule (e.g., a polypeptide or polynucleotide) is altered from its natural state by virtue of separating it from some or all of the naturally occurring constituents with which it is associated in nature. Such isolation or purification may be accomplished by art-recognized separation techniques such as ion exchange chromatography, affinity chromatography, hydrophobic separation, dialysis, protease treatment, ammonium sulphate precipitation or other protein salt precipitation, centrifugation, size exclusion chromatography, filtration, microfiltration, gel electrophoresis or separation on a gradient to remove whole cells, cell debris, impurities, extraneous proteins, or enzymes undesired in the final composition. It is further possible to then add constituents to a purified or isolated biomolecule composition which provide additional benefits, for example, activating agents, anti-inhibition agents, desirable ions, compounds to control pH or other enzymes or chemicals.

As used herein, the terms "enhanced", "improved" and "increased" when referring to expression of a biomolecule of interest (e.g., a protein on interest) are used interchangeably herein to indicate that expression of the biomolecule is above the level of expression in a corresponding host strain (e.g., a wildtype and/or a parental strain) that has not been altered according to the teachings herein but has been grown under essentially the same growth conditions.

As used herein the term "expression" when applied to a protein refers to a process by which a protein is produced based on the nucleic acid sequence of a gene and thus includes both transcription and translation.

As used herein in the context of introducing a polynucleotide into a cell, the term "introduced" refers to any method suitable for transferring the polynucleotide into the cell. Such methods for introduction include but are not limited to protoplast fusion, transfection, transformation, conjugation, and transduction (*See e.g.,* Ferrari et al., "Genetics," in Hardwood et al, (eds.), Bacillus, Plenum Publishing Corp., pages 57-72, [1989]).

As used herein, the terms "transformed" and "stably transformed" refers to a cell into which a polynucleotide sequence has been introduced by human intervention. The polynucleotide can be integrated into the genome of the cell or be present as an episomal plasmid that is maintained for at least two generations.

As used herein, the terms "selectable marker" or "selective marker" refer to a nucleic acid (*e.g.,* a gene) capable of expression in host cell which allows for ease of selection of those hosts containing the nucleic acid. Examples of such selectable markers include but are not limited to antimicrobials. Thus, the term "selectable marker" refers to genes that provide an indication that a host cell has taken up an incoming DNA of interest or some other reaction has occurred. Typically, selectable markers are genes that confer antimicrobial resistance or a metabolic advantage on the host cell to allow cells containing the exogenous DNA to be distinguished from cells that have not received any exogenous sequence during the transformation. Other markers useful in accordance with the invention include, but are not limited to auxotrophic markers, such as tryptophan; and detection markers, such as β-galactosidase.

As used herein, the term "promoter" refers to a nucleic acid sequence that functions to direct transcription of a downstream gene. In embodiments, the promoter is appropriate to the host cell in which the target gene is being expressed. The promoter, together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

As used herein, "functionally attached" or "operably linked" means that a regulatory region or functional domain having a known or desired activity, such as a promoter, terminator, signal sequence or enhancer region, is attached to or linked to a target (e.g., a gene or polypeptide) in such a manner as to allow the regulatory region or functional domain to control the expression, secretion or function of that target according to its known or desired activity.

The term "genetic alteration" or "genetic change" when used to describe a recombinant cell means that the cell has at least one genetic difference as compared to a parent cell. The one or more genetic difference may be a chromosomal mutation (e.g., an insertion, a deletion, substitution, inversion, replacement of a chromosomal region with another (e.g., replacement of a chromosomal prompter with a heterologous promoter), etc.) and/or the introduction of an extra-chromosomal polynucleotide (e.g., a plasmid). In some embodiments, an extra-chormosomal polynucleotide may be integrated into the chromosome of the host cell to generate a stable transfectant/transformant. Embodiments of the present disclosure include genetic alterations that reduce the expression of one or more genes in the *pdh* operon (*pdhA, pdhB, pdhC,* and *pdhD*). As detailed herein, such alterations improve the expression of a priteon of interest.

"Inactivation" of a gene means that the expression of a gene or the activity of its encoded biomolecule is blocked or is otherwise unable to exert its known function. Inactivation can occur via any suitable means, e.g., via a genetic alteration as described above. In one embodiment, the expression product of an inactivated gene is a truncated protein with a corresponding change in the biological activity of the protein. In some embodiments, an altered Gram positive bactarial strain comprises inactivation of one or more genes that results preferably in stable and non-reverting inactivation.

In some embodiments, inactivation is achieved by deletion. In some embodiments, the region targeted for deletion (e.g., a gene) is deleted by homologous recombination. For example, a DNA construct comprising an incoming sequence having a selective marker flanked on each side by sequences that are homologous to the region targeted for deletion is used (where the sequences may be referred to herein as a "homology box"). The DNA construct aligns with the homologous sequences of the host chromosome and in a double crossover event the region targeted fot deletion is excised out of the host chromosome.

An "insertion" or "addition" is a change in a nucleotide or amino acid sequence which has resulted in the addition of one or more nucleotides or amino acid residues, respectively, as compared to the naturally occurring or parental sequence.

As used herein, a "substitution" results from the replacement of one or more nucleotides or amino acids by different nucleotides or amino acids, respectively.

Methods of mutating genes are well known in the art and include but are not limited to site-directed mutation, generation of random mutations, and gapped-duplex approaches (*See e.g.,* U.S. Pat. 4,760,025; Moring et al., Biotech. 2:646 [1984]; and Kramer et al., Nucleic Acids Res., 12:9441 [1984]).

As used herein, "homologous genes" refers to a pair of genes from different, but usually related species, which correspond to each other and which are identical or very similar to each other. The term encompasses genes that are separated by speciation (*i.e.,* the development of new species) (e.g., orthologous genes), as well as genes that have been separated by genetic duplication (e.g., paralogous genes).

As used herein, "ortholog" and "orthologous genes" refer to genes in different species that have evolved from a common ancestral gene (*i.e.,* a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "homology" refers to sequence similarity or identity, with identity being preferred. This homology is determined using standard techniques known in the art (*See e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

As used herein, an "analogous sequence" is one wherein the function of the gene is essentially the same as the gene designated from *Bacillus subtilis* strain 168. Additionally, analogous genes include at least 60%, 65%, 70%, 75%, 80%, 85%, 90%, 95%, 97%, 98%, 99% or 100% sequence identity with the sequence of the *Bacillus subtilis* strain 168 gene. Alternately, analogous sequences have an alignment of between 70 to 100% of the genes found in the *B. subtilis* 168 region and/or have at least between 5 - 10 genes found in the region aligned with the genes in the *B*. *subtilis* 168 chromosome. In additional embodiments more than one of the above properties applies to the sequence. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps.

Another example of a useful algorithm is the BLAST algorithm, described by Altschul *et* al., (Altschul et al., J. Mol. Biol., 215:403-410, [1990]; and Karlin et al., Proc. Natl. Acad. Sci. USA 90:5873-5787 [1993]). A particularly useful BLAST program is the WU-BLAST-2 program (See, Altschul et al., Meth. Enzymol.,, 266:460-480 [1996]). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

As used herein, "percent (%) sequence identity" with respect to the amino acid or nucleotide sequences identified herein is defined as the percentage of amino acid residues or nucleotides in a candidate sequence that are identical with the amino acid residues or nucleotides in a Mal3A sequence, after aligning the sequences and introducing gaps, if necessary, to achieve the maximum percent sequence identity, and not considering any conservative substitutions as part of the sequence identity.

By "homologue" (or "homolog") shall mean an entity having a specified degree of identity with the subject amino acid sequences and the subject nucleotide sequences. A homologous sequence is can include an amino acid sequence that is at least 60%, 65%, 70%, 75%, 80%, 85%, 86%, 87%, 88%, 89%, 90%, 91%, 92%, 93%, 94%, 95%, 96%, 97%, 98% or even 99% identical to the subject sequence, using conventional sequence alignment tools (e.g., Clustal, BLAST, and the like). Typically, homologues will include the same active site residues as the subject amino acid sequence, unless otherwise specified.

Methods for performing sequence alignment and determining sequence identity are known to the skilled artisan, may be performed without undue experimentation, and calculations of identity values may be obtained with definiteness. See, for example, Ausubel et al., eds. (1995) Current Protocols in Molecular Biology, Chapter 19 (Greene Publishing and Wiley-Interscience, New York); and the ALIGN program (Dayhoff (1978) in Atlas of Protein Sequence and Structure 5:Suppl. 3 (National Biomedical Research Foundation, Washington, D.C.). A number of algorithms are available for aligning sequences and determining sequence identity and include, for example, the homology alignment algorithm of Needleman et al. (1970) J. Mol. Biol. 48:443; the local homology algorithm of Smith et al. (1981) Adv. Appl. Math. 2:482; the search for similarity method of Pearson et al. (1988) Proc. Natl. Acad. Sci. 85:2444; the Smith-Waterman algorithm (Meth. Mol. Biol. 70:173-187 (1997); and BLASTP, BLASTN, and BLASTX algorithms (see Altschul et al. (1990) J. Mol. Biol. 215:403-410).

Computerized programs using these algorithms are also available, and include, but are not limited to: ALIGN or Megalign (DNASTAR) software, or WU-BLAST-2 (Altschul et al., Meth. Enzym., 266:460-480 (1996)); or GAP, BESTFIT, BLAST, FASTA, and TFASTA, available in the Genetics Computing Group (GCG) package, Version 8, Madison, Wisconsin, USA; and CLUSTAL in the PC/Gene program by Intelligenetics, Mountain View, California. Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can determined by using Clustal W (Thompson J.D. et al. (1994) Nucleic Acids Res. 22:4673-4680) with default parameters, i.e.:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

As used herein, the term "hybridization" refers to the process by which a strand of nucleic acid joins with a complementary strand through base pairing, as known in the art.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5 °C (5° below the Tm of the probe); "high stringency" at about 5-10 °C below the Tm; "intermediate stringency" at about 10-20 °C below the Tm of the probe; and "low stringency" at about 20-25 °C below the Tm. Functionally, maximum stringency conditions may be used to identify sequences having strict identity or near-strict identity with the hybridization probe; while an intermediate or low stringency hybridization can be used to identify or detect polynucleotide sequence homologs.

Moderate and high stringency hybridization conditions are well known in the art. An example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37 °C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denaturated sheared salmon sperm DNA, followed by washing the filters in 1x SSC at about 37 - 50 °C. Those of skill in the art know how to adjust the temperature, ionic strength, etc. as necessary to accommodate factors such as probe length and the like.

The term "recombinant," when used in reference to a biological component or composition (e.g., a cell, nucleic acid, polypeptide/enzyme, vector, etc.) indicates that the biological component or composition is in a state that is not found in nature. In other words, the biological component or composition has been modified by human intervention from its natural state. For example, a recombinant cell encompass a cell that expresses one or more genes that are not found in its native parent (i.e., non-recombinant) cell, a cell that expresses one or more native genes in an amount that is different than its native parent cell, and/or a cell that expresses one or more native genes under different conditions than its native parent cell. Recombinant nucleic acids may differ from a native sequence by one or more nucleotides, be operably linked to heterologous sequences (e.g., a heterologous promoter, a sequence encoding a non-native or variant signal sequence, etc.), be devoid of intronic sequences, and/or be in an isolated form. Recombinant polypeptides/enzymes may differ from a native sequence by one or more amino acids, may be fused with heterologous sequences, may be truncated or have internal deletions of amino acids, may be expressed in a manner not found in a native cell (e.g., from a recombinant cell that over-expresses the polypeptide due to the presence in the cell of an expression vector encoding the polypeptide), and/or be in an isolated form. It is emphasized that in some embodiments, a recombinant polynucleotide or polypeptide/enzyme has a sequence that is identical to its wild-type counterpart but is in a non-native form (e.g., in an isolated or enriched form).

As used herein, the term "target sequence" refers to a DNA sequence in the host cell that encodes the sequence where it is desired for the incoming sequence to be inserted into the host cell genome. In some embodiments, the target sequence encodes a functional wild-type gene or operon, while in other embodiments the target sequence encodes a functional mutant gene or operon, or a non-functional gene or operon.

As used herein, a "flanking sequence" refers to any sequence that is either upstream or downstream of the sequence being discussed (*e.g.,* for genes A-B-C, gene B is flanked by the A and C gene sequences). In an embodiment, the incoming sequence is flanked by a homology box on each side. In another embodiment, the incoming sequence and the homology boxes comprise a unit that is flanked by stuffer sequence on each side. In some embodiments, a flanking sequence is present on only a single side (either 3' or 5'), but in embodiments, it is on each side of the sequence being flanked. The sequence of each homology box is homologous to a sequence in the *Bacillus* chromosome. These sequences direct where in the *Bacillus* chromosome the new construct gets integrated and what part of the *Bacillus* chromosome will be replaced by the incoming sequence. In an embodiment, the 5' and 3' ends of a selective marker are flanked by a polynucleotide sequence comprising a section of the inactivating chromosomal segment. In some embodiments, a flanking sequence is present on only a single side (either 3' or 5'), while in embodiments, it is present on each side of the sequence being flanked.

As used herein, the terms "amplifiable marker," "amplifiable gene," and "amplification vector" refer to a gene or a vector encoding a gene which permits the amplification of that gene under appropriate growth conditions.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (*See e.g.,* Kacian et al., Proc. Natl. Acad. Sci. USA 69:3038 [1972]). Other nucleic acids are not replicated by this amplification enzyme. Similarly, in the case of T7 RNA polymerase, this amplification enzyme has a stringent specificity for its own promoters (*See*, Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (*See*, Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "amplifiable nucleic acid" refers to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e.,* a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g.,* ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195 4,683,202, and 4,965,188 which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e.,* denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

With PCR, it is possible to amplify a single copy of a specific target sequence in genomic DNA to a level detectable by several different methodologies (*e.g.,* hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "RT-PCR" refers to the replication and amplification of RNA sequences. In this method, reverse transcription is coupled to PCR, most often using a one enzyme procedure in which a thermostable polymerase is employed, as described in U.S. Patent No. 5,322,770. In RT-PCR, the RNA template is converted to cDNA due to the reverse transcriptase activity of the polymerase, and then amplified using the polymerizing activity of the polymerase (*i.e.,* as in other PCR methods).

As used herein, "genetically altered host strain" (e.g., agenetically altered Bacillus strain) refers to a genetically engineered host cell, also called a recombinant host cell. In some embodiments, the genetically altered host cell has enhanced (increased) expression of a protein of interest as compared to the expression and/or production of the same protein of interest in a corresponding unaltered host strain grown under essentially the same growth conditions. In some embodiments, the enhanced level of expression results from reduced expression of one or more gene from the *pdh* operon. In some embodiments, the altered strains are genetically engineered *Bacillus* sp. having one or more deleted indigenous chromosomal regions or fragments thereof, wherein a protein of interest has an enhanced level of expression or production, as compared to a corresponding unaltered *Bacillus* host strain grown under essentially the same growth conditions.

As used herein, a "corresponding unaltered *Bacillus* strain" and the like is the host strain (*e.g.,* the originating (parental) and/or wild-type strain) which does not have the indicated genetic alteration.

As used herein, the term "chromosomal integration" refers to the process whereby the incoming sequence is introduced into the chromosome of a host cell (*e.g., Bacillus*). The homologous regions of the transforming DNA align with homologous regions of the chromosome. Subsequently, the sequence between the homology boxes is replaced by the incoming sequence in a double crossover (*i.e.,* homologous recombination). In some embodiments of the present invention, homologous sections of an inactivating chromosomal segment of a DNA construct align with the flanking homologous regions of the indigenous chromosomal region of the *Bacillus* chromosome. Subsequently, the indigenous chromosomal region is deleted by the DNA construct in a double crossover (*i.e.,* homologous recombination).

"Homologous recombination" means the exchange of DNA fragments between two DNA molecules or paired chromosomes at the site of identical or nearly identical nucleotide sequences. In a embodiment, chromosomal integration is homologous recombination.

"Homologous sequences" as used herein means a nucleic acid or polypeptide sequence having 100%, 99%, 98%, 97%, 96%, 95%, 94%, 93%, 92%, 91%, 90%, 88%, 85%, 80%, 75%, or 70% sequence identity to another nucleic acid or polypeptide sequence when optimally aligned for comparison. In some embodiments, homologous sequences have between 85% and 100% sequence identity, while in other embodiments there is between 90% and 100% sequence identity, and in more embodiments, there is 95% and 100% sequence identity.

As used herein "amino acid" refers to peptide or protein sequences or portions thereof. The terms "protein", "peptide" and "polypeptide" are used interchangeably.

As used herein, "protein of interest" and "polypeptide of interest" refer to a protein/polypeptide that is desired and/or being assessed. In some embodiments, the protein of interest is intracellular, while in other embodiments, it is a secreted polypeptide. Particularly polypeptides include enzymes, including, but not limited to those selected from amylolytic enzymes, proteolytic enzymes, cellulytic enzymes, oxidoreductase enzymes and plant cell-wall degrading enzymes. More particularly, these enzyme include, but are not limited to amylases, proteases, xylanases, lipases, laccases, phenol oxidases, oxidases, cutinases, cellulases, hemicellulases, esterases, perioxidases, catalases, glucose oxidases, phytases, pectinases, glucosidases, isomerases, transferases, galactosidases and chitinases. In particular embodiments of the present invention, the polypeptide of interest is a protease. In some embodiments, the protein of interest is a secreted polypeptide which is fused to a signal peptide (*i.e.,* an amino-terminal extension on a protein to be secreted). Nearly all secreted proteins use an amino- terminal protein extension which plays a crucial role in the targeting to and translocation of precursor proteins across the membrane. This extension is proteolytically removed by a signal peptidase during or immediately following membrane transfer.

In some embodiments of the present invention, the polypeptide of interest is selected from hormones, antibodies, growth factors, receptors, etc. Hormones encompassed by the present invention include but are not limited to, follicle-stimulating hormone, luteinizing hormone, corticotropin-releasing factor, somatostatin, gonadotropin hormone, vasopressin, oxytocin, erythropoietin, insulin and the like. Growth factors include, but are not limited to platelet-derived growth factor, insulin-like growth factors, epidermal growth factor, nerve growth factor, fibroblast growth factor, transforming growth factors, cytokines, such as interleukins (*e.g.,* IL-1 through IL-13), interferons, colony stimulating factors, and the like. Antibodies include but are not limited to immunoglobulins obtained directly from any species from which it is desirable to produce antibodies. In addition, the present invention encompasses modified antibodies. Polyclonal and monoclonal antibodies are also encompassed by the present invention. In particularly embodiments, the antibodies are human antibodies.

As used herein, a "derivative" or "variant" of a polypeptide means a polypeptide, which is derived from a precursor polypeptide (e.g., the native polypeptide) by addition of one or more amino acids to either or both the C- and N-terminal ends, substitution of one or more amino acids at one or a number of different sites in the amino acid sequence, deletion of one or more amino acids at either or both ends of the polypeptide or at one or more sites in the amino acid sequence, insertion of one or more amino acids at one or more sites in the amino acid sequence, and any combination thereof. The preparation of a derivative or variant of a polypeptide may be achieved in any convenient manner, e.g., by modifying a DNA sequence which encodes the native polypeptides, transformation of that DNA sequence into a suitable host, and expression of the modified DNA sequence to form the derivative/variant polypeptide. Derivatives or variants further include polypeptides that are chemically modified.

As used herein, the term "heterologous protein" refers to a protein or polypeptide that does not naturally occur in the host cell. Examples of heterologous proteins include enzymes such as hydrolases including proteases, cellulases, amylases, carbohydrases, and lipases; isomerases such as racemases, epimerases, tautomerases, or mutases; transferases, kinases and phophatases. In some embodiments, the proteins are therapeutically significant proteins or peptides, including but not limited to growth factors, cytokines, ligands, receptors and inhibitors, as well as vaccines and antibodies. In additional embodiments, the proteins are commercially important industrial proteins/peptides (*e.g.,* proteases, carbohydrases such as amylases and glucoamylases, cellulases, oxidases and lipases). In some embodiments, the gene encoding the proteins are naturally occurring genes, while in other embodiments, mutated and/or synthetic genes are used.

As used herein, "homologous protein" refers to a protein or polypeptide native or naturally occurring in a cell. In embodiments, the cell is a Gram-positive cell, while in particularly embodiments, the cell is a *Bacillus* host cell. In alternative embodiments, the homologous protein is a native protein produced by other organisms, including but not limited to *E. coli.* The invention encompasses host cells producing the homologous protein via recombinant DNA technology.

As used herein, an "operon" comprises a group of contiguous genes that can be transcribed as a single transcription unit from a common promoter, and are thereby subject to co-regulation. In some embodiments, an operon may include multiple promoters that drive the transcription of multiple different mRNAs (see, e.g., the promoters in the *phd* operon schematized in Figure 1).

The present invention relates in general to bacterial cells having a genetic alteration that results in increased expression of a protein of interest and methods of making and using such cells. Aspects of the present invention include Gram-positive microorganisms, such as *Bacillus* species, having a genetic alteration that reduces the expression of a gene in the *pdh* operon and results in enhanced expression of a protein of interest.

As summarized above, aspects of the invention include methods for increasing expression of a protein of interest from a Gram positive bacterial cell and is based on the observation that the production of a protein of interest is increased in Gram positive cells that have been genetically altered to have reduced expression of one or more genes in the *pdh* operon is as compared to the expression level of the same protein of interest in a corresponding non-genetically altered Gram positive cell (e.g., a wild type and/or a parental cell). By genetic alteration is meant any alteration in a host cell that changes the genetic make-up of the host cell, for example by episomal addition and/or chromosomal insertion, deletion, inversion, base change, etc. No limitation in this regard is intended.

In certain embodiments, the method involves producing or obtaining an altered Gram positive bacterial cell that comprises at least one genetic alteration that reduces expression of one or more genes in the *pdh* operon and that is capable of producing a protein of interest and culturing the altered Gram positive bacterial cell under conditions such that the protein of interest is expressed by the altered Gram positive bacterial cell. Expression of the protein of interest is thereby increased in the altered Gram positive bacterial cell compared to the expression of the protein of interest in a corresponding unaltered Gram positive bacterial cell grown under essentially the same culture conditions.

According to certain embodiments, the genetically altered Gram positive bacterial cell (or parental cell from which the genetically altered Gram positive bacterial cell is produced) can be a *Bacillus* strain. In some embodiments, the *Bacillus* strain of interest is alkalophilic. Numerous alkalophilic *Bacillus* strains are known (*See e.g.,* U.S. Pat. 5,217,878; and Aunstrup et al., Proc IV IFS: Ferment. Technol. Today, 299-305 [1972]). In some embodiments, the *Bacillus* strain of interest is an industrial *Bacillus* strain. Examples of industrial *Bacillus* strains include, but are not limited to *B. licheniformis, B. lentus, B. subtilis,* and *B. amyloliquefaciens.* In additional embodiments, the *Bacillus* host strain is selected from the group consisting of *B*. *lentus, B. brevis, B. stearothermophilus, B. alkalophilus, B. coagulans, B. circulans, B. pumilus, B. thuringiensis, B. clausii,* and *B. megaterium,* as well as other organisms within the genus *Bacillus,* as discussed above. In particular embodiments, *B. subtilis* is used. For example, U.S. Patents 5,264,366 and 4,760,025 (RE 34,606) describe various *Bacillus* host strains that find use in the present invention, although other suitable strains are contemplated for use in the present invention.

The parental strain of a genetically altered cell as diescribed herein (e.g., a parental *Bacillus* strain) may be an industrial strain, which includes non-recombinant strains, mutant strains of a naturally occurring strain, or a recombinant strain. In certain embodiments, the parental strain is a recombinant host strain wherein a polynucleotide encoding a polypeptide of interest has been introduced into the host. While the introduction of a polynucleotide encoding a polypeptide of interest may be done in a parental strain, this step may also be performed in a strain that has already been genetically altered for increased polypeptide production as detailed herein. In some embodiments, the host strain is *a Bacillus subtilis* host strain, e.g., a recombinant *B. subtilis* host strain.

Numerous *B. subtilis* strains are known that find use in aspects of the present invention, including but not limited to 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211 strain (*See e.g.,* Hoch et al., Genetics, 73:215-228 [1973]; U.S. Patent No. 4,450,235; U.S. Patent No. 4,302,544; and EP 0134048). The use of *B*. *subtilis* as an expression host is further described by Palva *et al.* and others (*See*, Palva et al., Gene 19:81-87 [1982]; *also see* Fahnestock and Fischer, J. Bacteriol., 165:796-804 [1986]; and Wang et al., Gene 69:39-47 [1988]).

In certain embodiments, industrial protease producing *Bacillus* strains can serve as parental expression hosts. In some embodiments, use of these strains in the present invention provides further enhancements in efficiency and protease production. Two general types of proteases are typically secreted by *Bacillus sp.,* namely neutral (or "metalloproteases") and alkaline (or "serine") proteases. Serine proteases are enzymes which catalyze the hydrolysis of peptide bonds in which there is an essential serine residue at the active site. Serine proteases have molecular weights in the 25,000 to 30,000 range (*See*, Priest, Bacteriol. Rev., 41:711-753 [1977]). Subtilisin is a serine protease for use in the present invention. A wide variety of *Bacillus* subtilisins have been identified and sequenced, for example, subtilisin 168, subtilisin BPN', subtilisin Carlsberg, subtilisin DY, subtilisin 147 and subtilisin 309 (*See e.g.,* EP 414279 B; WO 89/06279; and Stahl et al., J. Bacteriol., 159:811-818 [1984]). In some embodiments of the present invention, the *Bacillus* host strains produce mutant (*e.g.,* variant) proteases. Numerous references provide examples of variant proteases and reference (*See e.g.,* WO 99/20770; WO 99/20726; WO 99/20769; WO 89/06279; RE 34,606; U.S. Patent No. 4,914,031; U.S. Patent No. 4,980,288; U.S. Patent No. 5,208,158; U.S. Patent No. 5,310,675; U.S. Patent No. 5,336,611; U.S. Patent No. 5,399,283; U.S. Patent No. 5,441,882; U.S. Patent No. 5,482,849; U.S. Patent No. 5,631,217; U.S. Patent No. 5,665,587; U.S. Patent No. 5,700,676; U.S. Patent No. 5,741,694; U.S. Patent No. 5,858,757; U.S. Patent No. 5,880,080; U.S. Patent No. 6,197,567; and U.S. Patent No. 6,218,165).

It is noted here that the present invention is not limited to proteases as the protein of interest. Indeed, the present disclosure encompasses a wide variety of proteins of interest for which increased expression in the Gram positive cell is desired (detailed below).

In other embodiments, a strain for use in aspects of the present invention may have additional genetic alterations in other genes that provide beneficial phenotypes. For example, a *Bacillus* sp. that includes a mutation or deletion in at least one of the following genes, *degU, degS, degR* and *degQ* may be employed. In some embodiments, the mutation is in a *degU* gene, e.g., a *degU(Hy)32* mutation. (*See*, Msadek et al., J. Bacteriol., 172:824-834 [1990]; and Olmos et al., Mol. Gen. Genet., 253:562-567 [1997]). Thus, one example of a parental/genetically altered Gram positive strain that finds use in aspects of the present invention is a *Bacillus subtilis* cell carrying a *degU32(Hy)* mutation. In a further embodiment, the *Bacillus* host may include a mutation or deletion in *scoC4,* (*See,* Caldwell et al., J. Bacteriol., 183:7329-7340 [2001]); *spollE* (See, Arigoni et al., Mol. Microbiol., 31:1407-1415 [1999]); *oppA* or other genes of the *opp* operon (See, Perego et al., Mol. Microbiol., 5:173-185 [1991]). Indeed, it is contemplated that any mutation in the *opp* operon that causes the same phenotype as a mutation in the *oppA* gene will find use in some embodiments of the altered *Bacillus* strain of the present invention. In some embodiments, these mutations occur alone, while in other embodiments, combinations of mutations are present. In some embodiments, an altered *Bacillus* of the invention is obtained from a parental *Bacillus* host strain that already includes a mutation to one or more of the above-mentioned genes. In alternate embodiments, a previously genetically altered *Bacillus* of the invention is further engineered to include mutation of one or more of the above-mentioned genes.

As indicated above, expression of at least one gene of the *pdh* operon is reduced in the genetically altered Gram positive cell as compared to a wildtype and/or parental cell (growm under essentially the same conditions). This reduction of expression can be achieced in any convenient manner, and may be at the level of transcription, mRNA stability, translation, or may be due to the presence of a varation in one or more of the polypeptides produced from the *pdh* operon that reduces its activity (i.e., it is a "functional" reduction of expression based on activity of the polypeptide). As such, no limitation in the type of genetic alteration or the manner through which expression of at least one gene in the pdh operon is reduced is intended. For example, in some embodiments the genetic alteration in the Gram positive cell is one that alters one or more of promoters in the *pdh* operon resulting in reduced transcriptional activity. In certain embodiments, the alteration is a silent mutation in the *pdh* operon that results in reduced levels of mRNA transcript (e.g., as shown in the examples). Alternatively, the genetic alteration in the Gram positive cell can be one that alters a nucleotide in the *pdh* operon resulting in a transcript with reduced stability in the cell. In certain embodiments, more than one genetic alteration that reduces the expression of one or more genes in the *pdh* operon may be present in the genetically altered Gram positive cell.

In certain embodiments, the expression of the one or more genes in the pdh operon is reduced in the genetically altered Gram positive cell to about 3% of the level of expression in the wildtype and/or parental cell cultured under essentailly the same culture conditions, including about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 35%, about 40%, about 45%, about 50%, about 55%, about 60%, about 65%, about 70%, about 75%, or about 80%. As such, the range of reduction of expression of the one or more genes in the pdh operon can be from about 3% to about 80%, from about 4% to about 75%, from about 5% to about 70%, from about 6% to about 65%, from about 7% to about 60%, from about 8% to about 50%, from about 9% to about 45%, from about 10% to about 40%, from about 11% to about 35%, from about 12% to about 30%, from about 13% to about 25%, from about 14% to about 20%, etc. Any sub-range of expression within the ranges set forth above is contemplated.

In certain embodiments, the altered Gram positive bacterial cell has reduced expression of the *pdhA* gene, and/or the *pdhB* gene, and/or the pdhC gene, and/or the *pdhD* gene, or any combination thereof, as compared to the expression of these genes in a corresponding unaltered Gram positive bacterial cell grown under essentially the same culture conditions. In particular embodiments, the genetic alteration results in a decrease in the level of an mRNA transcript derived from the *pdh* operon in the altered Gram positive bacterial cell as compared to a corresponding un-altered Gram positive bacterial cell grown under essentially the same culture conditions.

In certain embodiments, the mutation is in the *pdhD* gene of the *pdh* operon. A *pdhD* gene in a parental Gram positive cell (i.e., prior to being genetically altered as described herein) is a gene that is at least 60% identical to SEQ ID NO:1, including at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to SEQ ID NO:1. In certain embodiments, the genetic alteration is a silent mulation, where by silent mutation is meant a mutation in the nucleic acid sequence of the coding region of a gene that does not result in an amino acid change in the encoded polypeptide whend translated (a term that is well understood in the art). In certain embodiments, the mutation is at a nucleotide position corresponding to nucleotide 729 of SEQ ID NO: 1. In certain embodiments, the mutation is a C to T mutation at a nucleotide position corresponding to nucleotide 729 of SEQ ID NO:1 (shown in SEQ ID NO:3).

As indicated above, many different proteins find use as the protein of interest in the Gram positive cell (i.e., the protein whose expression is increased in the genetically altered cell). The protein of interest can be a homologous protein or a heterologous protein and may be a wildtype protein or a natural or recombinant variant. In certain embodiments, the protein of interest is an enzyme, where in certain instances, the enzyme is selected from a protease, cellulase, pullulanase, amylase, carbohydrase, lipase, isomerase, transferase, kinase, and phosphatase. In certain embodiments, the protein of interest is a protease, where the protese may be a subtilisin, e.g., a subtilisin selected from subtilisin 168, subtilisin BPN', subtilisin Carlsberg, subtilisin DY, subtilisin 147, subtilisin 309, and variants thereof. In certain embododimetns, the protein of interest is a fluorescent protein, e.g., green fluorescent protein (GFP).

In certain embodiments, the method further comprisies recovering the protein of interest. Because the level of expression/production of the protein of interest is increased in the genetically altered Gram positive cell (as comparet to q wildtype or parental cell), the amount of the protein of interest recovered is increases as compared to the corresponding wildtype and/or parental cell cultured under essentiall the same culture conditions (and at the same scale). There are various assays known to those of ordinary skill in the art for detecting and measuring the expression level/production of intracellularly and extracellularly expressed polypeptides. Such assays will be determined by the user of the present invention and may depend on the identity and/or activity (e.g., enzymatic activity) of the protein of interest. For example, for proteases, there are assays based on the release of acid-soluble peptides from casein or hemoglobin measured as absorbance at 280 nm or colorimetrically using the Folin method (*See e.g.,* Bergmeyer et al., "Methods of Enzymatic Analysis" vol. 5, Peptidases, Proteinases and their Inhibitors, Verlag Chemie, Weinheim [1984]). Other assays involve the solubilization of chromogenic substrates (*See e.g.,* Ward, "Proteinases," in Fogarty (ed.)., Microbial Enzymes and Biotechnology, Applied Science, London, [1983], pp 251-317). Other examples of assays include succinyl-Ala-Ala-Pro-Phe-para nitroanilide assay (SAAPFpNA) and the 2,4,6-trinitrobenzene sulfonate sodium salt assay (TNBS assay). Numerous additional references known to those in the art provide suitable methods (*See e.g.,* Wells et al., Nucleic Acids Res. 11:7911-7925 [1983]; Christianson et al., Anal. Biochem., 223:119 -129 [1994]; and Hsia et al., Anal Biochem., 242:221-227 [1999]) .

Also as indicated above, means for determining the levels of secretion of a protein of interest in a host cell and detecting expressed proteins include the use of immunoassays with either polyclonal or monoclonal antibodies specific for the protein of interest. Examples include enzyme-linked immunosorbent assay (ELISA), radioimmunoassay (RIA), fluorescence immunoassay (FIA), and fluorescent activated cell sorting (FACS). However, other methods are known to those in the art and find use in assessing the protein of interest (*See e.g.,* Hampton et al., Serological Methods, A Laboratory Manual, APS Press, St. Paul, MN [1990]; and Maddox et al., J. Exp. Med., 158:1211 [1983]). As known in the art, the altered *Bacillus* cells produced using the present invention are maintained and grown under conditions suitable for the expression and recovery of a polypeptide of interest from cell culture (*See e.g.,* Hardwood and Cutting (eds.) Molecular Biological Methods for Bacillus, John Wiley & Sons [1990]). It is further noted that a genetically altered cell as described herein may express more than one protein of interest, including two or more, three or more, four or more, five or more, six or more, seven or more, eight or more, nine or more, ten or more, etc. In some embodiments, increased expression of proteins in the bacterial secretome is desired, which includes numerous different proteins that are secretred from the cell.

Aspects of the present invention include a method for obtaining an altered Gram positive bacterial cell with improved protein production capability. In general, the method includes genetically altering a parental Gram positive cell to result in a genetically altered strain in which the expression of one or more gene in the pdh operon is reduced (as defined above).

In certain embodiments, the method includes introducing a polynucleotide sequence into a parental Gram positive bacterial cell that, when integrated into the chromosome or sustained as an episomal genetic element, results in a genetically altered Gram positive cell in which the expression level of one or more genes in the *pdh* operon is reduced.

Various methods are known for the transformation of *Bacillus* species to alter the chromosome of, or to maintain an episomal genetic element in, *Bacillus* using polynucldotide vectors (e.g., plasmid constructs) are well known. Suitable methods for introducing polynucleotide sequences into *Bacillus* cells are found in, *e.g.,* Ferrari et al., "Genetics," in Harwood et al. (ed.), Bacillus, Plenum Publishing Corp. [1989], pages 57-72; *See also,* Saunders et al., J. Bacteriol., 157:718-726 [1984]; Hoch et al., J. Bacteriol., 93:1925 -1937 [1967]; Mann et al., Current Microbiol., 13:131-135 [1986]; and Holubova, Folia Microbiol., 30:97 [1985]; for *B*. *subtilis,* Chang et al., Mol. Gen. Genet., 168:11-115 [1979]; for *B*. *megaterium,* Vorobjeva et al., FEMS Microbiol. Lett., 7:261-263 [1980]; for *B amyloliquefaciens,* Smith et al., Appl. Env. Microbiol., 51:634 (1986); for *B*. *thuringiensis,* Fisher et al., Arch. Microbiol., 139:213-217 [1981]; and for *B*. *sphaericus,* McDonald, J. Gen. Microbiol.,130:203 [1984]. Indeed, such methods as transformation including protoplast transformation and congression, transduction, and protoplast fusion are known and suited for use in the present invention. Methods of transformation are particularly to introduce a DNA construct provided by the present invention into a host cell

In addition, introduction of a DNA construct into the host cell includes physical and chemical methods known in the art to introduce DNA into a host cell without insertion of the targeting DNA construct into a plasmid or vector. Such methods include, but are not limited to calcium chloride precipitation, electroporation, naked DNA, liposomes and the like. In additional embodiments, DNA constructs can be co-transformed with a plasmid, without being inserted into the plasmid.

In embodiments in which selectable marker genes are used to select for stble transformants, it may be desireable to delete the selective marker from the genetically altered Gram positive strain using any convenient method, with numerous methods being known in the art (See, Stahl et al., J. Bacteriol., 158:411-418 [1984]; and Palmeros et al., Gene 247:255 -264 [2000]).

In some embodiments, two or more DNA constructs (i.e., DNA constructs that each are designed to genetically alter a host cell) are introduced into a parental Gram positive cell, resulting in the introduction of two or more genetic alterations in the cell, e.g., alterations at two or more chromosomal regions. In some embodiments, these regions are contiguous, (*e.g.,* two regions within the pdh operon or within the pdh operon and an adjacent gene or operon), while in other embodiments, the regions are separated. In some embodiments, one or more of the genetic alterations are by addition of an episomal genetic element.

In some embodiments, host cells are transformed with one or more DNA constructs according to the present invention to produce an altered *Bacillus* strain wherein two or more genes have been inactivated in the host cell. In some embodiments, two or more genes are deleted from the host cell chromosome. In alternative embodiments, two or more genes are inactivated by insertion of a DNA construct. In some embodiments, the inactivated genes are contiguous (whether inactivated by deletion and/or insertion), while in other embodiments, they are not contiguous genes.

Once a genetically altered host cell is produced, it can be cultured under conditions such that the protein of interest is expressed, where in certain embodiments the protein of interest is recovered.

Aspects of the present invention include an altered Gram positive bacterial cell, wherein the altered Gram positive bacterial cell comprises at least one genetic alteration that reduces expression of one or more genes in the *pdh* operon as compared to a corresponding unaltered Gram positive bacterial cell grown under essentially the same culture conditions. In some embodiments, the genetically altered Gram positive cell is produced as described above. As further noted above, the altered Gram positive bacterial cell can be a *Bacillus sp.* strain, e.g., a *B*. *licheniformis, B. lentus, B. subtilis, B*. amyloliquefaciens, *B. brevis, B. stearothermophilus, B. alkalophilus, B. coagulans, B. circulans, B. pumilus, B. lautus, B. clausii, B. megaterium,* or *B*. *thuringiensis* strain. In certain embodiments, the *Bacillus sp.* strain is a *B*. *subtilis* strain. In some aspects, the altered Gram positive bacterial cell further comprises an additional mutation that improves a phenotype of the cell, e.g., a mutation in a gene selected from the group consisting of *degU, degQ, degS, scoC4, spollE,* and *oppA.* In certain embodiments, the mutation is *degU(Hy)32.*

In certain embodiments, expression of at least one gene of the *pdh* operon is reduced in the genetically altered Gram positive cell as compared to a wildtype and/or parental cell (growm under essentially the same conditions). This reduction of expression can be achieced in any convenient manner, and may be at the level of transcription, mRNA stability, translation, or may be due to the presence of a varation in one or more of the polypeptides produced from the *pdh* operon that reduces its activity (i.e., it is a "functional" reduction of expression based on activity of the polypeptide). As such, no limitation in the type of genetic alteration or the manner through which expression of at least one gene in the pdh operon is reduced is intended. For example, in some embodiments the genetic alteration in the Gram positive cell is one that alters one or more of promoters in the *pdh* operon resulting in reduced transcriptional activity. In certain embodiments, the alteration is a silent mutation in the *pdh* operon that results in reduced levels of mRNA transcript (e.g., as shown in the examples). Alternatively, the genetic alteration in the Gram positive cell can be one that alters a nucleotide in the *pdh* operon resulting in a transcript with reduced stability in the cell. In certain embodiments, more than one genetic alteration that reduces the expression of one or more genes in the *pdh* operon may be present in the genetically altered Gram positive cell. In certain embodiments, the genetic alteration results in a decrease in the level of an mRNA transcript derived from the *pdh* operon in the altered Gram positive bacterial cell as compared to a corresponding unaltered Gram positive bacterial cell grown under essentially the same culture conditions.

In some embodiments, the present invention includes a DNA construct comprising an incoming sequence that, when stably incorporated into the host cell, genetically alters the cell such that expression of one or more genes in the pdh operon is reduced (as described in detail above). In some emboeiments, the DNA construct is assembled *in vitro,* followed by direct cloning of the construct into a competent Gram positive (e.g., *Bacillus*) host such that the DNA construct becomes integrated into the host cell chromosome. For example, PCR fusion and/or ligation can be employed to assemble a DNA construct *in vitro.* In some embodiments, the DNA construct is a non-plasmid construct, while in other embodiments it is incorporated into a vector (*e.g.,* a plasmid). In some embodiments, circular plasmids are used. In embodiments, circular plasmids are designed to use an appropriate restriction enzyme (*i.e.,* one that does not disrupt the DNA construct). Thus, linear plasmids find use in the present invention. However, other methods are suitable for use in the present invention, as known to those in the art (*See e.g.,* Perego, "Integrational Vectors for Genetic Manipulation in Bacillus subtilis," in (Sonenshein et al. (eds.), Bacillus subtilis and Other Gram-Positive Bacteria, American Society for Microbiology, Washington, DC [1993]).

In certain embodiments, the incoming sequence of a DNA targeting vector incudes a polynucleotide comprising a variant sequence derived from the *pdhD* gene. In some of these embodiments, the variant sequence is at least about 30 nucleotides in length, is at least 95% identical to all or a part of SEQ ID NO:1, and has at least one mutation at a nucleotide position in the *pdhD* gene that leads to reduced expression of a gene in the *pdh* operon when the mutation is present in the endogenous *pdhD* gene of a Gram positive bacterial cell. The variant sequence can be at least about 30 nucleotides, about 40 nucleotides, about 50 nucleotides, about 60 nucleotides, about 80 nucleotides, about 90 nucleotides, about 100 nucleotides, about 200 nucleotides, about 300 nucleotides, about 400 nucleotides, about 500 nucleotides, about 600 nucleotides, about 700 nucleotides, about 800 nucleotides, about 900 nucleotides, about 1000 nucleotides, about 1100 nucleotides, about 1200 nucleotides, about 1300 nucleotides, about 1400 or more nucleotides. The variant sequence can be at least about 85%, at least about 90%, at least about 91%, at least about 92%, at least about 93%, at least about 94%, at least about 95%, at least about 96%, at least about 97%, at least about 98%, or at least about 99% identical to SEQ ID NO:1. In certain embodiments, the genetic alteration in the variant sequence is a silent mulation, where by silent mutation is meant a mutation in the variant sequence of the coding region of the *pdhD* gene that does not result in an amino acid change in the encoded PdhD polypeptide when translated (a term that is well understood in the art). In certain embodiments, the mutation in the variant sequence is at a nucleotide position corresponding to nucleotide 729 of SEQ ID NO: 1. In certain embodiments, the mutation in the variant sequence is a C to T mutation at a nucleotide position corresponding to nucleotide 729 of SEQ ID NO:1 (shown in SEQ ID NO:3).

Aspects of the present invention include a vector comprising the polynucleotide sequence as described above. In certain embodiments, the vector is a targeting vector designed to introduce the at least one mutation in the polynucleotide sequence into the corresponding location in the *pdh* operon of a Gram positive bacterial cell by homologous recombination when transformed into the Gram positive bacterial cell. In some embodiments, the incoming sequence/vector includes a selective marker. In some embodiment, the selective marker located between two *lox*P sites (See, Kuhn and Torres, Meth. Mol. Biol.,180:175-204 [2002]), and the antimicrobial gene is then deleted by the action of Cre protein.

Aspects of the present invention include a method for enhancing expression of a protein of interest in a Gram positive bacterial cell that includes transforming a parental Gram positive bacterial cell with the DNA construct or vector described above (i.e., one that includes an incoming sequence that, when stably incorporated into the host cell, genetically alters the cell such that expression of one or more genes in the *pdh* operon is reduced, e.g., one that includes a mutation in the pdhD gene as set forth above), allowing homologous recombination of the vector and the corresponding region in the *pdh* operon of the parental Gram positive bacterial cell to produce an altered Gram positive bacterial cell; and growing the altered Gram positive bacterial cell under conditions suitable for the expression of the protein of interest, where the production of the protein of interest is increased in the altered Gram positive bacterial cell as compared to the Gram positive bacterial cell prior to the transformation in step. Examples of the Gram positive strains, mutations and other features that find use in this aspect of the invention are described in detail above.

Whether the DNA construct is incorporated into a vector or used without the presence of plasmid DNA, it is used to transform microorganisms. It is contemplated that any suitable method for transformation will find use with the present invention. In embodiments, at least one copy of the DNA construct is integrated into the host *Bacillus* chromosome. In some embodiments, one or more DNA constructs of the invention are used to transform host cells.

The manner and method of carrying out the present invention may be more fully understood by those of skill in the art by reference to the following examples, which examples are not intended in any manner to limit the scope of the present invention or of the claims directed thereto.

### EXPERIMENTAL

The following Examples are provided in order to demonstrate and further illustrate certain embodiments and aspects of the present invention and are not to be construed as limiting the scope thereof.

In the experimental disclosure which follows, certain of the following abbreviations apply: °C (degrees Centigrade); rpm (revolutions per minute); µg (micrograms); mg (milligrams); µl (microliters); ml (milliliters); mM (millimolar); µM (micromolar); sec (seconds); min(s) (minute/minutes); hr(s) (hour/hours); OD₂₈₀ (optical density at 280 nm); OD₆₀₀ (optical density at 600 nm); PCR (polymerase chain reaction); RT-PCR (reverse transcription PCR); SDS (sodium dodecyl sulfate).

### EXAMPLE 1

### Increased protein expression in Bacillus by mutation in the pdh operon

### A. The pdh operon

Figure 1 shows a schematic diagram of the pdh operon of *Bacillus subtilis.* In Figure 1, each coding region in the *pdh* operon is indicated with an arrow that indicates the direction of transcription (*pdhA, pdhB, pdhC* and *pdhD*)*.* In addition to the coding regions, transcriptional start sites are shown in bent arrows. As indicatd on Figure 1, transcription of the *pdhA* and *pdhB* coding regions is thought to be controlled by a SigA promoter present before the *pdhA* gene. Similarly, the *pdhC* and *pdhD* genes are thought to be controlled by a SigA promoter present before the *pdhC* gene. In addition to the SigA promoter in fromt of the *pdhC* gene, the transcription of the *pdhD* gene may also be controlled by its own promoter in addition to the SigA promoter in front of the *pdhC* gene (shown as a bent arrow with a question mark in front of the *pdhD* gene). While this schematic represents our current understanding regarding the control of transcription the pdh operon, it is not meant to be comprehensive. As such, other promoter/transcription factor interactions that are not shown in Figure 1 may be involved in controlling the transcription of genes in the *pdh* operon.

The genes in the *pdh* operon shown in Figure 1 are as follows:
1. PdhA (Pyruvate dehydrogenase E1 component, alpha subunit);
2. PdhB (Pyruvate dehydrogenase E1 component, beta subunit);
3. PdhC (Pyruvate dehydrogenase [dihydrolipoamide acetyltransferase E2 subunit]); and
4. PdhD (Pyruvate dehydrogenase/2-oxoglutarate dehydrogenase [dihydrolipoamide dehydrogenase E3 subunit]).

Of the genes listed above, *pdhA* is considered to be an essential gene. The *pdhD* gene is involved in the recycling of pyruvate in the Acetyl-CoA pathway and is the is the last gene of the *pdh* operon: (*pdhAB)-pdhCD.*

### B. Mutation of the pdhD gene in the pdh operon

A silent mutation was introduced into parental *Bacillus subtilis* strain CB15-14 (*amyE::xylR*P*xylAcomK-ermC,* Δ*oppA* Δ*spoIIE,* Δ*aprE,* Δ*nprE, degUHy32,* Δ*scoC*) in the *pdhD* gene of the *pdh* operon using the method described by Janes and Stibitz (Infection and Immunity, 74(3):1949, 2006). The *pdhD* silent mutation is a single nucleotide change from cytosine (C) to thymine (T) at nucleotide position 729 of the sense strand of the coding sequence of wildtype *pdhD* (SEQ ID NO:1 shows the wildtype sequence; SEQ ID NO:3 shows the C729T silent mutation) (a silent mutation is a mutation that changes the nucleic acid sequence of a site in the coding region of a gene but does not change the amino acid sequence of the encoded polypeptide). The resultant strain CB15-14 *pdhD* is sometimes referred to herein as "the *pdhD* mutated strain", "the mutant strain", or equivalents thereof. The non-mutated strain (CB15-14) is sometimes refered to herein as "the parental strain" or equivalents thereof.

### C. Amylase expression in the pdhD mutant strain

An amylase expression construct which drives the expression of AmyE (mature sequence shown in SEQ ID NO:4) from the aprE promoter and which includes a chloramphenico acetyltransferase resistance (catR) marker gene (denoted P*aprE-amyE catR*) was introduced into the *aprE* locus of the *pdhD* mutated strain and the non-mutated parental strain. The strains were amplified on Luria agar plates containing 25 µg/ml of chloramphenicol. The *pdhD* mutated strain and the wild type strain were grown overnight in 5 mL of Luria broth medium. 1 ml of pre-culture was used to inoculate 25 ml of Luria broth medium in shake flasks at 37°C, 250 rpm to test the expression of the *amyE* amylase gene. Cell densities were measured at 600 nm at hourly intervals using a SpectraMax spectrophotometer (Molecular Devices, Downington, PA, USA). The absorbance at 600 nm was plotted as a function of time and the results are shown in Figure 2A. Figure 2A shows that the cell growth of the AmyE expressing parental strain CB15-14 and the AmyE expressing CB15-14 *pdh* strain is equivalent, indicating that the presence of the *pdhD* mutation in the CB15-14 *pdh* strain does not affect the cell growth.

AmyE amylase activity of whole broth was measured using the Ceralpha reagent (Megazyme, Wicklow, Ireland.). The Ceralpha reagent mix from the Ceralpha HR kit was initially dissolved in 10 ml of MilliQ water followed by the addition of 30 ml of 50 mM malate buffer, pH 5.6. The culture supernatants were diluted 40X in MilliQ water and 5 µl of diluted sample was added to 55µL of diluted working substrate solution. The MTP plate was incubated for 4 minutes at room temperature after shaking. The reaction was quenched by adding 70 µl of 200 mM borate buffer pH 10.2 (stop solution). The absorbance of the solution was measured at 400 nm using a SpectraMax spectrophotometer (Molecular Devices, Downington, PA, USA). The absorbance at 400 nm was plotted as a function of time and the results are shown in Figure 2B. The graph in Figure 2B shows increased AmyE production starting at 6 hours of growth in the CB15-14 *pdh* mutant strain. Given that cell growth was not affected in the mutant strain (as shown in Figure 2A), the increase in AmyE production in Bacillus cells having the pdhD mutation as compared to the parental cells grown under the same culture conditions is not due to an increase in the number of cells in the culture, but rather due to increased expression levels in the cells themselves (i.e., on a cell-by-cell basis).

### D. Protease (FNA) expression in the pdhD mutant strain

To test the effect of the silent mutation in the *pdhD* gene on expression of FNA protease (subtilisin BPN' containing the Y217L substitution; SEQ ID NO:5), the *PaprE-FNA catR* construct (expresses FNA from the aprE promoter and includes a chloramphenico acetyltransferase resistance (catR) marker gene) was introduced in the *aprE* locus of the CB15-14 parental strain and the CB15-14 *pdhD* mutant strain. The *PaprE-FNA catR* construct was amplified as described in (C) above. Two clones were analyzed for each (clones 1 and 2 for the parental FNA cells and clones 1 and 18 for mutant cells). The *pdhD* mutated strains and the wild type strains were grown overnight in 5 mL of Luria broth. 1 ml of pre-culture was used to inoculate 25 ml of 2XNB (2X Nutrient Broth, 1XSNB salts, described in WO2010/14483) in Thompson flasks at 250 rpm to test protease expression. Cell densities of whole broth diluted 20X were measured at 600 nm at hourly intervals using a SpectraMax spectrophotometer (Molecular Devices, Downington, PA, USA). The absorbance at 600nm was plotted as a function of time and the results are shown in Figure 3A. Figure 3A shows that the cell growth of the FNA expressing parental strain CB15-14 is reduced as compared to the FNA expressing CB15-14 *pdh* strain, indicating that the presence of the *pdhD* mutation in these FNA expressing strains positively affects cell growth.

Protease expression was monitored using N-suc-AAPF-pNA substrate (from Sigma Chemical Co.) as described in WO 2010/144283. Briefly, whole broth was diluted 40X in the assay buffer (100 mM Tris, 0.005% Tween 80, pH 8.6) and 10 µl of the diluted samples were arrayed in microtiter plates. The AAPF stock was diluted and the assay buffer (100 X dilution of 100 mg/ml AAPF stock in DMSO) and 190 µl of this solution were added to the microtiter plates and the absorbance of the solution was measured at 405 nm using a SpectraMax spectrophotometer (Molecular Devices, Downington, PA, USA). The absorbance at 405 nm was plotted as a function of time and the results are shown in Figure 3B. As shown in Figure 3B, FNA production is increased in Bacillus cell cultures having the pdhD mutation as compared to cultures of the parental cells grown under the same culture conditions. The increased FNA production may be due to an increase in the number of cells present in the *pdhD* mutant cell culture as compared to the parental strain culture.

### E. Green Fluorescent Protein (GFP) expression in the pdhD mutant strain

To test the effect of the silent mutation in the *pdhD* gene on expression of other proteins, the *PaprE-GFP catR* construct, which has GFP expression controlled by the aprE promoter and includes a chloramphenicol acetyltransrefase resistance marker (SEQ ID NO:6 shows the amino acid sequence of GFP), was introduced in the *aprE* locus of the CB15-14 parental strain and CB15-14 *pdhD* mutant strain. Transformants were selected on Luria agar plates containing 5µg/ml of chloramphenicol. Two *pdhD* mutated strains expressing GFP (clones 1 and 2) and two wild type strains expressing GFP (clones 1 and 2) were grown overnight in 5 mL of Luria broth. 1 ml of pre-culture was used to inoculate 25 ml of 2XNB medium (2X nutrient broth, 1X SNB salts) in shake flasks at 37°C, 250 rpm to test the expression of green fluorescent protein (GFP). Cell densities of whole broth diluted 20X were measured at 600 nm at hourly intervals using a SpectraMax spectrophotometer (Molecular Devices, Downington, PA, USA). The absorbance at 600 nm was plotted as a function of time and the results are shown in Figure 4A. Upon the entry into stationary phase (between 4 and 6 hrs of growth in 2XNB), the decline in the cell growth in the pdhD mutant strains is delayed compared the control strains, indicating improved cell viability due to the *pdhD* mutation.

To measure GFP expression, 100 µl of culture was transferred to a 96 well microtiter plate and GFP expression was measured in a fluorescent plate reader using an excitation wavelength of 485 nm, an emission wavelength of 508 nm with a 495 nm emission cutoff filter. The relative fluorescence units (RFU) at 485/508 nm were plotted as a function of time and the results are shown in Figure 4B. The graph shows an increased of GFP production from 6 hrs of growth due to the *pdhD* mutation. The level of increased GFP expression in the mutant strain as compared to the wildtype strain exceeds what would be expected merely from the improvement in cell viability seen in Figure 4A.

### F. Beta-D-glucosidase (BgIC) expression in the pdhD mutant strain

To test the effect of the silent mutation in the *pdhD* gene on beta-D-glucosidase (BgIC) expression (SEQ ID NO:7 shows the amino acid sequence of BgIC), the *PaprE-BgIC catR* construct, which has BgIC expression controlled by the aprE promoter and includes a chloramphenicol acetyltransrefase resistance marker, was introduced in the *aprE* locus of the CB15-14 parental strain and CB15-14 *pdhD* mutant strain. Transformants were selected on Luria agar plates containing 5µg/ml of chloramphenicol. Two *pdhD* mutated strains (clones 1 and 2) and two wild type strains (clones 1 and 2) expressing BgIC were grown overnight in 5 mL of Luria broth. 1 ml of pre-culture was used to inoculate 25 ml of 2XNB medium (2X nutrient broth, 1X SNB salts) in shake flasks at 37°C, 250 rpm to test the expression of the secreted BgIC. Cell densities of whole broth diluted 20X were measured at 600 nm at hourly intervals using a SpectraMax spectrophotometer (Molecular Devices, Downington, PA, USA). The absorbance at 600 nm was plotted as a function of time and the results are shown in Figure 5A. Similar cell densities have been found for the control strains and the derivative strains containing the pdhD mutation.

BgIC expression was monitored using 4-Nitrophenyl-β-D- cellobioside substrate (Sigma Chemicals, St. Louis, MO, USA, Cat. #N57590). The substrate was dissolved in 1 ml of DMSO to create the stock solution at 100 mg/ml. The working substrate solution was made by diluting 35 µl of the stock solution in 10 ml of assay buffer (100 mM Tris, 0.005% Tween 80, pH 8.6). Forty microliters of each culture was transferred to a 96 well microtiter plate and 180 µl of the working substrate solution was added to each well. The microtiter plate was incubated at room temperature for 5 hours and at the end of the incubation period, the absorbance of the solution was measured at 405 nm using a SpectraMax spectrophotometer (Molecular Devices, Downington, PA, USA). The absorbance at 405 nm was plotted as a function of time and the results are shown in Figure 5B. The graph shows an increased level of expression of BgIC from the mutant strain as compared to the parental strain under the same culture conditions throughout the time course experiment. Given that cell growth was not affected in the mutant strain (as shown in Figure 5A), the increase in BgIC production in Bacillus cells having the pdhD mutation as compared to the parental cells grown under the same culture conditions is not due to an increase in the number of cells in the culture, but rather due to increased expression levels in the cells themselves (i.e., on a cell-by-cell basis).

### G. mRNA transcripts of pdhA and pdhB genes are reduced in the pdhD mutated strain

A quantitative RT-PCR analysis was performed to determine the effect of the silent mutation in the *pdhD* gene on the mRNA transcript levels of the *pdhA* and *pdhB* genes. Total RNA was extracted from the CB15-14 parental strain and the *pdhD* mutant strain expressing FNA in 2XNB at the 4hr time point (cells in exponential growth phase; cells are from experiment performed as in (D) above) and treated with dsDNase (ArcticZymes). A real time RT-PCR experiment was performed using the Roche LightCycler LC 480 (Roche Diagnostics Corp., IN, USA) and the LNA (Locked Nucleic Acid) probes (Roche) to compare the amount of *pdhA* and *pdhB* transcripts in the wild-type and mutant strains. The *pdhA* and *pdhB* mRNAs were amplified using the following oligomers and universal probes (UPL):

### pdhA:

UPL probe 148
oligo 644: 5'- agctatcgttgacagtaagaagca-3' (SEQ ID NO:8)
oligo 645: 5'-ttggaacgtttcgaactgc-3' (SEQ ID NO:9)

### pdhB

UPL probe 136
Oligo 646: atcatcacttacggcgcaat UPL (SEQ ID NO:10)
Oligo 647: tcagcagaaatgccgtctt UPL (SEQ ID NO:11)

The Light Cycler 480 software (15.0) was used to determine the fractional cycle number (Crossing point, Cp) and to quantify the transcripts amounts. The absolute quantities of of *pdhA* and *pdhB* transcripts were calculated by the exponential of the fractional cycle numbers for each sample (log₂[Cp]). The amounts of *pdhA* and *pdhB* transcripts in the parental strain CB15-14 were compared to the transcript amounts in the *pdhD* mutant strain.

The relative amounts of *pdhA* and *pdhB* transcripts in the CB15-14 and the *pdhD* mutant strains are shown in Table 1.

**Table 1: Relative amounts of pdhA and pdhB transcripts from CB15-14 (parental) and CB15-14-pdhD mutant strains**

| | CB15-14 | CB15-14 pdhD | |
|---|---|---|---|
| ***pdhA*** | 1 | 0.171 | |
| ***pdhB*** | 1 | 0.285 | |

Table 1 shows that the amount of *pdhA* and *pdhB* transcript is significantly lower in the pdhD mutant strains than in the parental strains.

In view of the data described above, it is clear that a reduction in expression from the *pdh* operon (e.g., the *pdhA* and/or *pdhB* gene) in a Gram positive bacterial cell (i.e., as compared to a parental cell) results in increased expression of a protein of interest as compared to the parental cell when cultured under the same, or essentially the same, culture conditions.

Furthermore, all examples and conditional language recited herein are principally intended to aid the reader in understanding the principles of the present compositions and methods and the concepts contributed by the inventors to furthering the art, and are to be construed as being without limitation to such specifically recited examples and conditions. Moreover, all statements herein reciting principles, aspects, and embodiments of the present compositions and methods as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present compositions and methods, therefore, is not intended to be limited to the embodiments shown and described herein.

### SEQUENCES

SEQ ID NO:1 - pdhD wildtype coding sequence, sense strand
ATGGTAGTAGGAGATTTCCCTATTGAAACAGATACTCTTGTAATTGGTGCGGGACCTGGCG
GCTATGTAGCTGCCATCCGCGCTGCACAGCTTGGACAAAAAGTAACAGTCGTTGAAAAAG
CAACTCTTGGAGGCGTTTGTCTGAACGTTGGATGTATCCCTTCAAAAGCGCTGATCAATGC
AGGTCACCGTTATGAGAATGCAAAACATTCTGATGACATGGGAATCACTGCTGAGAATGTA
ACAGTTGATTTCACAAAAGTTCAAGAATGGAAAGCTTCTGTTGTCAACAAGCTTACTGGCG
GTGTAGCAGGTCTTCTTAAAGGCAACAAAGTAGATGTTGTAAAAGGTGAAGCTTACTTTGT
AGACAGCAATTCAGTTCGTGTTATGGATGAGAACTCTGCTCAAACATACACGTTTAAAAAC
GCAATCATTGCTACTGGTTCTCGTCCTATCGAATTGCCAAACTTCAAATATAGTGAGCGTGT
CCTGAATTCAACTGGCGCTTTGGCTCTTAAAGAAATTCCTAAAAAGCTCGTTGTTATCGGC
GGCGGATACATCGGAACTGAACTTGGAACTGCGTATGCTAACTTCGGTACTGAACTTGTTA
TTCTTGAAGGCGGAGATGAAATTCTTCCTGGCTTCGAAAAACAAATGAGTTCTCTCGTTAC
ACGCAGACTGAAGAAAAAGGCAACGTTGAAATCCATACAAACGCGATGGCTAAAGGCGT
TGAAGAAAGACCAGACGGCGTAACAGTTACTTTCGAAGTAAAAGGCGAAGAAAAAACTGTT
GATGCTGATTACGTATTGATTACAGTAGGACGCCGTCCAAACACTGATGAGCTTGGTCTTG
AGCAAGTCGGTATCGAAATGACGGACCGCGGTATCGTGAAAACTGACAAACAGTGCCGCA
CAAACGTACCTAACATTTATGCAATCGGTGATATCATCGAAGGACCGCCGCTTGCTCATAA
AGCATCTTACGAAGGTAAAATCGCTGCAGAAGCTATCGCTGGAGAGCCTGCAGAAATCGA
TTACCTTGGTATTCCTGCGGTTGTTTTCTCTGAGCCTGAACTTGCATCAGTTGGTTACACTG
AAGCACAGGCGAAAGAAGAAGGTCTTGACATTGTTGCTGCTAAATTCCCATTTGCAGCAAA
CGGCCGCGCGCTTTCTCTTAACGAAACAGACGGCTTCATGAAGCTGATCACTCGTAAAGA
GGACGGTCTTGTGATCGGTGCGCAAATCGCCGGAGCAAGTGCTTCTGATATGATTTCTGA
ATTAAGCTTAGCGATTGAAGGCGGCATGACTGCTGAAGATATCGCAATGACAATTCACGCT
CACCCAACATTGGGCGAAATCACAATGGAAGCTGCTGAAGTGGCAATCGGAAGTCCGATT
CACATCGTAAAATAA

SEQ ID NO:2 - pdhD protein sequence
MVVGDFPIETDTLVIGAGPGGYVAAIRAAQLGQKVTVVEKATLGGVCLNVGCIPSKALINAGHR
YENAKHSDDMGITAENVTVDFTKVQEWKASVVNKLTGGVAGLLKGNKVDVVKGEAYFVDSNS
VRVMDENSAQTYTFKNAIIATGSRPIELPNFKYSERVLNSTGALALKEIPKKLVVIGGGYIGTELG

TAYANFGTELVILEGGDEILPGFEKQMSSLVTRRLKKKGNVEIHTNAMAKGVEERPDGVTVTFE
VKGEEKTVDADYVLITVGRRPNTDELGLEQVGIEMTDRGIVKTDKQCRTNVPNIYAIGDIIEGPP
LAHKASYEGKIAAEAIAGEPAEIDYLGIPAVVFSEPELASVGYTEAQAKEEGLDIVAAKFPFAAN
GRALSLNETDGFMKLITRKEDGLVIGAQIAGASASDMISELSLAIEGGMTAEDIAMTIHAHPTLGE
ITMEAAEVAIGSPIHIVK

SEQ ID NO:3 - pdhD mutant coding sequence, sense strand (with C729T silent mutation)
ATGGTAGTAGGAGATTTCCCTATTGAAACAGATACTCTTGTAATTGGTGCGGGACCTGGCG
GCTATGTAGCTGCCATCCGCGCTGCACAGCTTGGACAAAAAGTAACAGTCGTTGAAAAAG
CAACTCTTGGAGGCGTTTGTCTGAACGTTGGATGTATCCCTTCAAAAGCGCTGATCAATGC
AGGTCACCGTTATGAGAATGCAAAACATTCTGATGACATGGGAATCACTGCTGAGAATGTA
ACAGTTGATTTCACAAAAGTTCAAGAATGGAAAGCTTCTGTTGTCAACAAGCTTACTGGCG
GTGTAGCAGGTCTTCTTAAAGGCAACAAAGTAGATGTTGTAAAAGGTGAAGCTTACTTTGT
AGACAGCAATTCAGTTCGTGTTATGGATGAGAACTCTGCTCAAACATACACGTTTAAAAAC
GCAATCATTGCTACTGGTTCTCGTCCTATCGAATTGCCAAACTTCAAATATAGTGAGCGTGT
CCTGAATTCAACTGGCGCTTTGGCTCTTAAAGAAATTCCTAAAAAGCTCGTTGTTATCGGC
GGCGGATACATCGGAACTGAACTTGGAACTGCGTATGCTAACTTCGGTACTGAACTTGTTA
TTCTTGAAGGCGGAGATGAAATTCTTCCTGGCTTCGAAAAACAAATGAGTTCTCTCGTTAC
ACGCAGACTGAAGAAAAAGGCAACGTTGAAATCCATACAAACGCGATGGCTAAAGGTGT
TGAAGAAAGACCAGACGGCGTAACAGTTACTTTCGAAGTAAAAGGCGAAGAAAAAACTGTT
GATGCTGATTACGTATTGATTACAGTAGGACGCCGTCCAAACACTGATGAGCTTGGTCTTG
AGCAAGTCGGTATCGAAATGACGGACCGCGGTATCGTGAAAACTGACAAACAGTGCCGCA
CAAACGTACCTAACATTTATGCAATCGGTGATATCATCGAAGGACCGCCGCTTGCTCATAA
AGCATCTTACGAAGGTAAAATCGCTGCAGAAGCTATCGCTGGAGAGCCTGCAGAAATCGA
TTACCTTGGTATTCCTGCGGTTGTTTTCTCTGAGCCTGAACTTGCATCAGTTGGTTACACTG
AAGCACAGGCGAAAGAAGAAGGTCTTGACATTGTTGCTGCTAAATTCCCATTTGCAGCAAA
CGGCCGCGCGCTTTCTCTTAACGAAACAGACGGCTTCATGAAGCTGATCACTCGTAAAGA
GGACGGTCTTGTGATCGGTGCGCAAATCGCCGGAGCAAGTGCTTCTGATATGATTTCTGA
ATTAAGCTTAGCGATTGAAGGCGGCATGACTGCTGAAGATATCGCAATGACAATTCACGCT
CACCCAACATTGGGCGAAATCACAATGGAAGCTGCTGAAGTGGCAATCGGAAGTCCGATT
CACATCGTAAAATAA

SEQ ID NO:4 - AmyE protein sequence
L T A P S I K S G T I L H A W N W S F N T L K H N M K D I H D A G Y T A I Q T S P I N Q V K
E G N Q G D K S M S N W Y W L Y Q P T S Y Q I G N R Y L G T E Q E F K E M C A A A E E
Y G I K V I V D A V I N H T T S D Y A A I S N E V K S I P N W T H G N T Q I K N W S D R W

D V T Q N S L L G L Y D W N T Q N T Q V Q S Y L K R F L D R A L N D G A D G F R F D A A
K H I E L P D D G S Y G S Q F W P N I T N T S A E F Q Y G E I L Q D S A S R D A A Y A N Y
M D V T A S N Y G H S I R S A L K N R N L G V S N I S H Y A S D V S A D K L V T W V E S H
D T Y A N D D E E S T W M S D D D I R L G W A V I A S R S G S T P L F F S R P E G G G N
G V R F P G K S Q I G D R G S A L F E D Q A I T A V N R F H N V M A G Q P E E L S N P N
G N N Q I F M N Q R G S H G V V L A N A G S S S V S I N T A T K L P D G R Y D N K A G A
G S F Q V N D G K L T G T I N A R S V A V L Y P D

SEQ ID NO:5 - FNA protein sequence (with pro-domain)
A Q S V P Y G V S Q I K A P A L H S Q G Y T G S N V K V A V I D S G I D S S H P D L K V A
G G A S M V P S E T N P F Q D N N S H G T H V A G T V A A L N N S I G V L G V A P S A S
L Y A V K V L G A D G S G Q Y S W I I N G I E W A I A N N M D V I N M S L G G P S G S A A
L K A A V D K A V A S G V V V V A A A G N E G T S G S S S T V G Y P G K Y P S V I A V G
A V D S S N Q R A S F S S V G P E L D V M A P G V S I Q S T L P G N K Y G A L N G T S M
A S P H V A G A A A L I L S K H P N W T N T Q V R S S L E N T T T K L G D S F Y Y G K G L
I N V Q A A A Q

SEQ ID NO:6 - GFP protein sequence
V N R N V L K N T G L K E I M S A K A S V E G I V N N H V F S M E G F G K G N V L F G N
Q L M Q I R V T K G G P L P F A F D I V S I A F Q Y G N R T F T K Y P D D I A D Y F V Q S F
P A G F F Y E R N L R F E D G A I V D I R S D I S L E D D K F H Y K V E Y R G N G F P S N
G P V M Q K A I L G M E P S F E V V Y M N S G V L V G E V D L V Y K L E S G N Y Y S C H
M K T F Y R S K G G V K E F P E Y H F I H H R L E K T Y V E E G S F V E Q H E T A I A Q L
T T I G K P L G S L H E W V

SEQ ID NO:7 - BglC protein sequence
A A G T K T P V A K N G Q L S I K G T Q L V N R D G K A V Q L K G I S S H G L Q W Y G E
Y V N K D S L K W L R D D W G I T V F R A A M Y T A D G G Y I D N P S V K N K V K E A V
E A A K E L G I Y V I I D W H I L N D G N P N Q N K E K A K E F F K E M S S L Y G N T P N
V I Y E I A N E P N G D V N W K R D I K P Y A E E V I S V I R K N D P D N I I I V G T G T W
S Q D V N D A A D D Q L K D A N V M Y A L H F Y A G T H G Q F L R D K A N Y A L S K G A
P I F V T E W G T S D A S G N G G V F L D Q S R E W L K Y L D S K T I S W V N W N L S D
K Q E S S S A L K P G A S K T G G W R L S D L S A S G T F V R E N I L G T K D S T K D I P
E T P S K D K P T Q E N G I S V Q Y R A G D G S M N S N Q I R P Q L Q I K N N G N T T V
D L K D V T A R Y W Y K A K N K G Q N F D C D Y A Q I G C G N V T H K F V T L H K P K Q

G A D T Y L E L G F K N G T L A P G A S T G N I Q L R L H N D D W S N Y A Q S G D Y S F
F K S N T F K T T K K I T L Y D Q G K L I W G T E P N

SEQ ID NO: 8 - oligo 644 for pdhA: agctatcgttgacagtaagaagca

SEQ ID NO: 9 - oligo 645 for pdhA: ttggaacgtttcgaactgc

SEQ ID NO: 10 - Oligo 646 for pdhB: atcatcacttacggcgcaat

SEQ ID NO: 11 - Oligo 647 for pdhB: tcagcagaaatgccgtctt

## Claims

1. A method for increasing expression of a protein of interest from a Gram positive bacterial cell comprising:
a) obtaining an altered Gram positive bacterial cell capable of producing a protein of interest, wherein said altered Gram positive bacterial cell comprises at least one genetic alteration that reduces expression of the *pdhA* gene as compared to the expression of the *pdhA* gene in a corresponding unaltered Gram positive bacterial cell grown under essentially the same culture conditions and/or reduces expression of the *pdhB* gene as compared to the expression of the *pdhB* gene in a corresponding unaltered Gram positive bacterial cell grown under essentially the same culture conditions wherein said genetic alteration is in the *pdhD* gene of the *pdh* operon, wherein the *pdhD* gene is at least 75% identical to SEQ ID NO: 1, and wherein said genetic alteration is at a nucleotide position corresponding to nucleotide 729 of SEQ ID NO: 1; and
b) culturing said altered Gram positive bacterial cell under conditions such that said protein of interest is expressed by said altered Gram positive bacterial cell, wherein expression of said protein of interest is increased in said altered Gram positive bacterial cell compared to the expression of said protein of interest in a corresponding unaltered Gram positive bacterial cell grown under essentially the same culture conditions.

2. An altered Gram positive bacterial cell, wherein said altered Gram positive bacterial cell comprises at least one genetic alteration that reduces expression of the *pdhA* gene as compared to the expression of the *pdhA* gene in a corresponding unaltered Gram positive bacterial cell grown under essentially the same culture conditions, wherein said genetic alteration is in the *pdhD* gene of the *pdh* operon and wherein said mutation is at a nucleotide position corresponding to nucleotide 729 of SEQ ID NO: 1.

3. The method of Claim 1 or the altered Gram positive bacterial cell of claim 2, wherein said altered Gram positive bacterial cell is a *Bacillus sp.* strain.

4. The method or the altered Gram positive bacterial cell of any one of Claims 1 to 3, wherein said altered Gram positive bacterial cell has reduced expression of the *pdhA* gene and the *pdhB* gene as compared to the expression of the *pdhA* gene and the *pdhB* gene in a corresponding unaltered Gram positive bacterial cell grown under essentially the same culture conditions.

5. The method or the altered Gram positive bacterial cell of any one of Claims 1 to 4, wherein said genetic alteration results in a decrease in the level of an mRNA transcript derived from the *pdh* operon in the altered Gram positive bacterial cell as compared to a corresponding unaltered Gram positive bacterial cell grown under essentially the same culture conditions.

6. The method or the altered Gram positive bacterial cell of any one of Claims 1 to 5, wherein said *pdhD* gene is at least 60% identical to SEQ ID NO: 1, optionally wherein said genetic alteration is a silent mutation.

7. The method or the altered Gram positive bacterial cell of any one of Claims 1 to 6, wherein said mutation is a C to T mutation at a nucleotide position corresponding to nucleotide 729 of SEQ ID NO: 1.

8. The altered Gram positive bacterial cell of any one of Claims 2 to 7, wherein said altered cell expresses a protein of interest.

9. The method of any one of Claims 1 or 3 to 7 or the altered Gram positive bacterial cell of claim 8, wherein said protein of interest is an enzyme, optionally wherein said protein of interest is a protease.

10. The method of any one of Claims 1, 3 to 7 and 9, further comprising recovering said protein of interest.

11. A polynucleotide comprising a variant sequence derived from the *pdhD* gene, wherein said variant sequence is:
(a) at least 30 nucleotides in length and at least 95% identical to all or a part of SEQ ID NO: 1, or
(b) at least 100 nucleotides in length and at least 90% identical to all or a part of SEQ ID NO: 1; or
(c) at least 500 nucleotides in length and at least 85% identical to all or a part of SEQ ID NO: 1;
and comprises at least one mutation at a nucleotide position in the *pdhD* gene that leads to reduced expression of the *pdhA* gene and/or the *pdhB* gene when said at least one mutation is present in the endogenous *pdhD* gene of a Gram positive bacterial cell, wherein said at least one mutation is a silent mutation at a nucleotide position corresponding to nucleotide 729 of SEQ ID NO: 1, wherein said mutation is a C to T mutation at a nucleotide position corresponding to nucleotide 729 of SEQ ID NO: 1.

12. A vector comprising the polynucleotide sequence of Claim 11, optionally wherein said vector is a targeting vector designed to introduce the at least one mutation in said polynucleotide sequence into the corresponding location in the *pdh* operon of a Gram positive bacterial cell by homologous recombination when transformed into said Gram positive bacterial cell.

13. A method for enhancing expression of a protein of interest in a Gram positive bacterial cell comprising:
a) transforming a parental Gram positive bacterial cell with the vector of Claim 12;
b) allowing homologous recombination of said vector and the corresponding region in the *pdh* operon of said parental Gram positive bacterial cell to produce an altered Gram positive bacterial cell; and
c) growing said altered Gram positive bacterial cell under conditions suitable for the expression of said protein of interest, wherein the production of said protein of interest is increased in the altered Gram positive bacterial cell as compared to said Gram positive bacterial cell prior to said transformation in step.

## Patentansprüche

1. Verfahren zum Erhöhung der Expression eines Proteins von Interesse von einer grampositiven Bakterienzelle, umfassend:
a) Erhalten einer veränderten grampositiven Bakterienzelle, die in der Lage ist, ein Protein von Interesse zu produzieren, wobei die veränderte grampositive Bakterienzelle mindestens eine genetische Veränderung umfasst, die die Expression des *pdhA*-Gens im Vergleich zu der Expression des *pdhA*-Gens in einer nicht veränderten grampositiven Bakterienzelle reduziert, die im Wesentlichen unter den gleichen Kulturbedingungen aufgezogen worden ist, und/oder die Expression des *pdhB-*Gens im Vergleich zu der Expression des *pdhB-*Gens in einer nicht veränderten grampositiven Bakterienzelle reduziert, die im Wesentlichen unter den gleichen Kulturbedingungen aufgezogen worden ist, wobei sich die genetische Veränderung in dem *pdhD-*Gen des *pdh*-Operon befindet, wobei das pdhD-Gen zu mindestens 75% identisch ist mit SEQ ID NO: 1, und wobei sich die genetische Veränderung an einer Nucleotid-Position befindet, die dem Nucleotid 729 von SEQ ID NO: 1 entspricht; und
b) Inkulturnehmen der veränderten grampositiven Bakterienzelle unter solchen Bedingungen, dass das Protein von Interesse durch die veränderten grampositiven Bakterienzelle exprimiert wird, wobei die Expression des Proteins von Interesse in der veränderten grampositiven Bakterienzelle im Vergleich zu der Expression des Proteins von Interesse in einer entsprechenden nicht veränderten grampositiven Bakterienzelle erhöht ist, die im Wesentlichen unter gleichen Kulturbedingungen aufgezogen worden ist.

2. Veränderte grampositive Bakterienzelle, wobei die veränderte grampositive Bakterienzelle mindestens eine genetische Veränderung umfasst, die die Expression des pdhA-Gens im Vergleich zu der Expression des *pdhA*-Gens in einer nicht veränderten grampositiven Bakterienzelle reduziert, die im Wesentlichen unter gleichen Kulturbedingungen aufgezogen worden ist, wobei sich die genetische Veränderung in dem *pdhD*-Gen des *pdh*-Operon befindet, und wobei sich die Mutation an einer Nucleotid-Position befindet, die dem Nucleotid 729 von SEQ ID NO: 1 entspricht.

3. Verfahren nach Anspruch 1, oder die veränderte grampositive Bakterienzelle von Anspruch 2, wobei die veränderte grampositive Bakterienzelle ein *Bacillus sp.* Stamm ist.

4. Verfahren oder die veränderte grampositive Bakterienzelle nach einem der Ansprüche 1 bis 3, wobei die veränderte grampositive Bakterienzelle reduzierte Expression des pdhA-Gens und des *pdhB-*Gens im Vergleich zu der Expression des *pdhA*-Gens und des *pdhB-*Gens in einer entsprechenden nicht veränderten grampositive Bakterienzelle aufweist, die im Wesentlichen unter gleichen Kulturbedingungen aufgezogen worden ist.

5. Verfahren oder die veränderte grampositive Bakterienzelle nach einem der Ansprüche 1 bis 4, wobei die genetische Veränderung zu einer Abnahme in dem Wert eines mRNA Transkripts resultiert, das von dem *pdh*-Operon in der veränderten grampositive Bakterienzelle deriviert ist, verglichen mit einer nicht veränderten grampositiven Bakterienzelle, die unter im Wesentlichen den gleichen Kulturbedingungen aufgezogen worden ist.

6. Verfahren oder die veränderte grampositive Bakterienzelle nach einem der Ansprüche 1 bis 5, wobei das pdhD-Gen zu mindestens 60% identisch ist mit SEQ ID NO: 1, wahlweise wobei die genetische Veränderung eine stille Mutation ist.

7. Verfahren oder die veränderte grampositive Bakterienzelle nach einem der Ansprüche 1 bis 6, wobei die Mutation eine Mutation C zu T an der Nucleotid-Position ist, die dem Nucleotid 729 von SEQ ID NO: 1 entspricht.

8. Veränderte grampositive Bakterienzelle nach einem der Ansprüche 2 bis 7, wobei die veränderte Zelle ein Protein von Interesse exprimiert.

9. Verfahren nach einem der Ansprüche 1 oder 3 bis 7 oder die veränderte grampositive Bakterienzelle nach Anspruch 8, wobei das Protein von Interesse ein Enzym ist, wahlweise wobei das Protein von Interesse eine Protease ist.

10. Verfahren nach einem der Ansprüche 1, 3 bis 7 und 9, ferner umfassend Gewinnen des Proteins von Interesse.

11. Polynucleotid, umfassend eine Sequenzvariante, die von dem *pdhD*-Gen deriviert ist, wobei die Sequenzvariante:
(a) eine Länge von mindestens 30 Nucleotiden aufweist und mindestens zu 95% identisch ist mit der gesamten oder einem Teil der SEQ ID NO: 1, oder
(b) eine Länge von mindestens 100 Nucleotiden aufweist und mindestens zu 90% identisch ist mit der gesamten oder einem Teil der SEQ ID NO: 1; oder
(c) eine Länge von mindestens 500 Nucleotiden aufweist und mindestens zu 85% identisch ist mit der gesamten oder einem Teil der SEQ ID NO: 1;
und mindestens eine Mutation an einer Nucleotid-Position in dem *pdhD*-Gen umfasst, die zu reduzierter Expression des *pdhA*-Gens und/oder des *pdhB-*Gens führt, wenn die mindestens eine Mutation in dem endogenen *pdhD*-Gen einer grampositiven Bakterienzelle vorhanden ist, wobei die mindestens eine Mutation eine stille Mutation an einer Nucleotid-Position ist, die dem Nucleotid 729 von SEQ ID NO: 1 entspricht, wobei die Mutation eine Mutation C zu T an einer Nucleotid-Position ist, die dem Nucleotid 729 von SEQ ID NO: 1 entspricht.

12. Vektor, umfassend die Polynucleotidsequenz nach Anspruch 11, wahlweise wobei der Vektor ein Zielvektor ist, der konzipiert ist, um die mindestens eine Mutation in der Polynucleotidsequenz an dem entsprechenden Ort in das *pdh*-Operon einer grampositiven Bakterienzelle durch homologe Rekombination einzuführen, wenn sie in die grampositive Bakterienzelle transformiert wird.

13. Verfahren zum Erhöhen der Expression eines Proteins von Interesse in einer grampositiven Bakterienzelle, umfassend:
a) Transformieren einer parentalen grampositiven Bakterienzelle mit dem Vektor nach Anspruch 12;
b) Ermöglichen einer homologen Rekombination des Vektors und der entsprechenden Region in dem *pdh*-Operon der parentalen grampositiven Bakterienzelle, um eine veränderte grampositive Bakterienzelle zu erzeugen; und
c) Aufziehen der veränderten grampositiven Bakterienzelle unter Bedingungen, die für die Expression des Proteins von Interesse geeignet sind, wobei die Produktion des Proteins von Interesse in der veränderten grampositiven Bakterienzelle im Vergleich zu der grampositiven Bakterienzelle vor der genannten Transformation im Schritt erhöht ist.

## Revendications

1. Procédé pour augmenter l'expression d'une protéine d'intérêt à partir d'une cellule bactérienne Gram positif, comprenant:
a) l'obtention d'une cellule bactérienne Gram positif modifiée capable de produire une protéine d'intérêt, laquelle cellule bactérienne Gram positif modifiée comprend au moins une modification génétique qui réduit l'expression du gène *pdhA* par rapport à l'expression du gène *pdhA* dans une cellule bactérienne Gram positif non modifiée correspondante développée essentiellement dans les mêmes conditions de culture et/ou réduit l'expression du gène *pdhB* par rapport à l'expression du gène *pdhB* dans une cellule bactérienne Gram positif non modifiée correspondante développée essentiellement dans les mêmes conditions de culture, dans laquelle ladite modification génétique est dans le gène *pdhD* de l'opéron *pdh,* dans laquelle le gène *pdhD* est au moins 75% identique à SEQ ID NO: 1, et dans laquelle ladite modification génétique est à une position de nucléotide correspondant au nucléotide 729 de SEQ ID NO: 1; et
b) la culture de ladite cellule bactérienne Gram positif modifiée dans des conditions telles que ladite protéine d'intérêt est exprimée par ladite cellule bactérienne Gram positif modifiée, dans laquelle l'expression de ladite protéine d'intérêt est augmentée dans ladite cellule bactérienne Gram positif modifiée par rapport à l'expression de ladite protéine d'intérêt dans une cellule bactérienne Gram positif non modifiée correspondante développée essentiellement dans les mêmes conditions de culture.

2. Cellule bactérienne Gram positif modifiée, laquelle cellule bactérienne Gram positif modifiée comprend au moins une modification génétique qui réduit l'expression du gène *pdhA* par rapport à l'expression du gène *pdhA* dans une cellule bactérienne Gram positif non modifiée correspondante développée essentiellement dans les mêmes conditions de culture, dans laquelle ladite modification génétique est dans le gène *pdhD* de l'opéron *pdh* et dans laquelle ladite mutation est à une position de nucléotide correspondant au nucléotide 729 de SEQ ID NO: 1.

3. Procédé selon la revendication 1 ou cellule bactérienne Gram positif modifiée selon la revendication 2, dans lesquels ladite cellule bactérienne Gram positif modifiée est une souche de *Bacillus sp.*

4. Procédé ou cellule bactérienne Gram positif modifiée selon l'une quelconque des revendications 1 à 3, dans lesquels ladite cellule bactérienne Gram positif modifiée a une expression réduite du gène *pdhA* et du gène *pdhB* par rapport à l'expression du gène *pdhA* et du gène *pdhB* dans une cellule bactérienne Gram positif non modifiée correspondante développée essentiellement dans les mêmes conditions de culture.

5. Procédé ou cellule bactérienne Gram positif modifiée selon l'une quelconque des revendications 1 à 4, dans lesquels ladite modification génétique conduit à une diminution du taux d'un produit de transcription d'ARNm issu de l'opéron *pdh* dans la cellule bactérienne Gram positif modifiée par rapport à une cellule bactérienne Gram positif non modifiée correspondante développée essentiellement dans les mêmes conditions de culture.

6. Procédé ou cellule bactérienne Gram positif modifiée selon l'une quelconque des revendications 1 à 5, dans lesquels ledit gène *pdhD* est au moins 60% identique à SEQ ID NO: 1, facultativement dans lesquels ladite modification génétique est une mutation silencieuse.

7. Procédé ou cellule bactérienne Gram positif modifiée selon l'une quelconque des revendications 1 à 6, dans lesquels ladite mutation est une mutation C à T à une position de nucléotide correspondant au nucléotide 729 de SEQ ID NO: 1.

8. Cellule bactérienne Gram positif modifiée selon l'une quelconque des revendications 2 à 7, laquelle cellule modifiée exprime une protéine d'intérêt.

9. Procédé selon l'une quelconque des revendications 1 ou 3 à 7 ou cellule bactérienne Gram positif modifiée selon la revendication 8, dans lesquels ladite protéine d'intérêt est une enzyme, facultativement dans lesquels ladite protéine d'intérêt est une protéase.

10. Procédé selon l'une quelconque des revendications 1, 3 à 7 et 9, comprenant en outre la récupération de ladite protéine d'intérêt.

11. Polynucléotide comprenant une séquence de variant issue du gène *pdhD,* dans lequel ladite séquence de variant est:
(a) d'au moins 30 nucléotides en longueur et au moins 95% identique à tout ou partie de SEQ ID NO: 1, ou
(b) d'au moins 100 nucléotides en longueur et au moins 90% identique à tout ou partie de SEQ ID NO: 1; ou
(c) d'au moins 500 nucléotides en longueur et au moins 85% identique à tout ou partie de SEQ ID NO: 1;
et comprend au moins une mutation à la position de nucléotide dans le gène *pdhD* qui conduit à une expression réduite du gène *pdhA* et/ou du gène *pdhB,* lorsque ladite au moins une mutation est présente dans le gène *pdhD* endogène d'une cellule bactérienne Gram positif, dans lequel ladite au moins une mutation est une mutation silencieuse à une position de nucléotide correspondant au nucléotide 729 de SEQ ID NO: 1, dans lequel ladite mutation est une mutation C à T à une position de nucléotide correspondant au nucléotide 729 de SEQ ID NO: 1.

12. Vecteur comprenant la séquence polynucléotidique selon la revendication 11, facultativement ledit vecteur étant un vecteur de ciblage élaboré pour introduire ladite au moins une mutation dans ladite séquence polynucléotidique dans l'emplacement correspondant de l'opéron *pdh* d'une cellule bactérienne Gram positif par recombinaison homologue, lorsqu'il est transformé dans ladite cellule bactérienne Gram positif.

13. Procédé pour augmenter l'expression d'une protéine d'intérêt dans une cellule bactérienne Gram positif, comprenant:
a) la transformation d'une cellule bactérienne Gram positif parente avec le vecteur selon la revendication 12;
b) le fait de permettre à la recombinaison homologue dudit vecteur et de la région correspondante dans l'opéron *pdh* de ladite cellule bactérienne Gram positif parente de produire une cellule bactérienne Gram positif modifiée; et
c) le développement de ladite cellule bactérienne Gram positif modifiée dans des conditions propices à l'expression de ladite protéine d'intérêt, dans lequel la production de ladite protéine d'intérêt est augmentée dans la cellule bactérienne Gram positif modifiée par rapport à ladite cellule bactérienne Gram positif avant ladite transformation dans l'étape.
